# EUROPEAN PATENT APPLICATION

(11) **EP 2 455 453 A1**
(43) Date of publication of application: **23.05.2012**
(21) Application number: 12151000.2
(22) Date of filing: 05.09.2007
(51) Int. Cl.: C12N 5/00, A61L 27/16, D01D 5/00, D01F 6/14, D01F 6/22, D01F 1/10, B82Y 5/00

(54) **Substrate for immobilizing cells or tissue**

(30) Priority: 06.09.2006 US 87244106 P; 15.03.2007 US 91808307 P
(62) Divisional of application: 07837733.0
(71) Applicant: Corning Incorporated, Corning, New York 14831 (US)
(72) Inventor: Baca, Adra S, Rochester, NY New York 14610 (US); Bryhan, Marie D, Lindley, NY New York 14858 (US); Noll, Frederick E, Horseheads, NY New York 14845 (US); Petzold, Odessa N, Elmira, NY New York 14901 (US); Price, Michael W, Corning, NY New York 14830 (US); Senaratne, Wageesha, Horseheads, NY New York 14845 (US); Walczak, Wanda J, Big Flats, NY New York 14814 (US)
(74) Representative: Oldroyd, Richard Duncan

(57) **Abstract**

Described herein are compositions for making nanofibers and nanofilms composed of metal oxides, organic polymers, or combinations thereof. Also described herein are methods for making nanofibers and nanofilms where the fibers are formed from a solution having more than one solvent, where the solvents are immiscible. Nanofibers and nanofilms composed of metal oxides, organic polymers or combinations thereof are described. Finally, methods for using the nanofibers and nanofilms are described.

## Description

### CROSS-REFERENCE TO RELATED APPLICATIONS

This application claims the benefit of U.S. Provisional Application Serial No. 60/872,441 filed on September 6, 2006 and U.S. Provision Application Serial No. 60/918,083 filed on March 15, 2007, which are incorporated by reference herein.

### BACKGROUND

The preparation and use of nanofibers and thin films has been studied extensively. Nanofibers and other thin films have numerous applications as supports or substrates. For example, nanofibers or nanofilms can be used as supports for catalysts. In other embodiments, nanofibers or nanofilms can be used as substrates for culturing cells. The ability to proliferate and differentiate cell growth *in vivo* has numerous applications. Cell growth *in vivo* occurs in the extracellular matrix (ECM), which is a three-dimensional environment. A two-dimensional surface such as a Petri dish surface for example, is not representative of cells growing *"in vivo."* Thus, in the case of nanofibers, it is possible to produce three-dimensional structures that simulate the extracellular matrix.

Depending upon the end-use of the substrate, it is desirable to modify the morphology of the nanofiber or nanofilm. For example, when the nanofiber or nanofilm is used to culture cells, it is desirable that the material have the proper porosity, surface area, and pore structure. Therefore, there is a need to be able to control the morphology of a nanofiber or nanofilm yet have the flexibility to use a wide variety of different starting materials to produce the nanofibers or nanofilms. The methods described herein provide a convenient way to modify the morphology of a nanofiber or nanofilm based on the selection of solvents used to prepare the solutions of starting materials prior to fiber- or film-formation.

### SUMMARY

In accordance with the purposes of the disclosed materials, compounds, compositions, articles, devices, and methods, as embodied and broadly described herein are nanofibers and nanofilms composed of metal oxides, organic polymers, or combinations thereof. Also described herein are compositions and methods for making nanofibers and nanofilms, which includes controlling the surface morphology of the fibers and films. Finally, methods for using the nanofibers and nanofilms are described. Additional advantages will be set forth in part in the description that follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

The accompanying figures, which are incorporated in and constitute a part of this specification, illustrate several aspects described below.
Figure 1 shows SEMs taken at 5000 x of Nb/PS with and without DMSO as well as Ta/PS electrospun nanofibers.
Figure 2 shows SEMs of Nb/PS and Ta/PS nanofibers showing outer texture and organic/inorganic phase mixing of nanofibers, where white arrows indicate regions of the inorganic phase.
Figure 3 shows proliferation for both HepG2 cell line and HMSCs on various electrospun substrates.
Figure 4 shows total protein production and cell proliferation on several nanofibers substrates compared to tissue culture treated lysine.
Figure 5 shows protein production as a function of surface charge as measured by zeta potential at a pH 7.0 in 1mM KCl.
Figure 6 shows albumin production as a function of substrate and nanofiber diameter.
Figure 7 shows albumin production as a function of substrate and nanofiber diameter normalized for cell count determined at the same time as albumin was assayed.
Figure 8 shows the complex surface morphology of a titania/polystyrene nanofiber composed of groove-like structures and pores.
Figure 9 shows additional surface features and the internal porosity, which has a surface area on the order of 14m²/gram.
Figure 10 shows a photomicrograph of the HEK293 cells attached to titania/polystyrene nanofibers.
Figure 11 shows the increase in cell counts on titania/polystyrene nanofibers compared to the normal polystyrene surface.
Figure 12 shows the more rounded shape of the HEK293 cells attached to the titania/polystyrene nanofibers.
Figure 13 shows SEMs of silica/PVA (59% silica) with typical nanofiber diameters around 200-400 nm at two different magnifications.
Figure 14 shows SEMs of 50/50 Sibrid™/PS nanofibers.
Figure 15 shows SEMs of 25/75 Sibrid™/PS nanofibers.
Figure 16 shows SEMs of a thermally cross-linkable silicone system Gelest OE43 part A and part B/PS silicone composition 10%.
Figure 17 shows optical micrographs of 50/50 and 25/75 sibrid™/PS hybrid electrospun mats.
Figure 18 shows MRC5 cells counts normalized to an area of substrate.
Figure 19 shows MRC5 cells on silica/PVA.
Figure 20 shows MRC5 cells on N₂O treated Sibrid™/PS surfaces.
Figure 21 shows HepG2 cells on sibrid™/PS hybrid electrospun mats (50/50, 25/75) that were either N₂O plasma treated (PL) or untreated (NO PL) or were less fused (LF) or more fused (MF).
Figure 22 shows HepG2 cells on silicone/PS hybrid electrospun mats.
Figure 23 shows HepG2 cells on silica/PVA hybrid electrospun mats with 59% silica composition with and without DMSO in mixture.
Figure 24 shows HepG2 cells on cross-linked silica/PVA hybrid electrospun mats with 59% silica composition.
Figure 25 shows albumin production as a function of substrate.
Figure 26 shows albumin production as a function of substrate normalized for cell count determined at the same time as albumin was assayed.
Figure 27 shows cell growth results on several nanofiber surfaces.
Figure 28 shows cell growth and protein production on several nanofiber surfaces.
Figure 29 is a graph showing the boiling point and vapor pressure of several solvents.

### DETAILED DESCRIPTION

The materials, compounds, compositions, articles, devices, and methods described herein may be understood more readily by reference to the following detailed description of specific aspects of the disclosed subject matter and the Examples included therein and to the Figures.

Before the present materials, compounds, compositions, articles, devices, and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

Also, throughout this specification, various publications are referenced. The disclosures of these publications in their entireties are hereby incorporated by reference into this application in order to more fully describe the state of the art to which the disclosed matter pertains. The references disclosed are also individually and specifically incorporated by reference herein for the material contained in them that is discussed in the sentence in which the reference is relied upon.

Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions, reference to "an agent" includes mixtures of two or more such agents, reference to "the layer" includes mixtures of two or more such layers, and the like.
"Optional" or "optionally" means that the subsequently described event or circumstance can or cannot occur, and that the description includes instances where the event or circumstance occurs and instances where it does not.

Certain materials, compounds, compositions, and components disclosed herein can be obtained commercially or readily synthesized using techniques generally known to those of skill in the art. For example, the starting materials and reagents used in preparing the disclosed compounds and compositions are either available from commercial suppliers or prepared by methods known to those skilled in the art.

Also, disclosed herein are materials, compounds, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a composition is disclosed and a number of modifications that can be made to a number of components of the composition are discussed, each and every combination and permutation that are possible are specifically contemplated unless specifically indicated to the contrary. Thus, if a class of components A, B, and C are disclosed as well as a class of components D, E, and F and an example of a composition A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific aspect or combination of aspects of the disclosed methods, and that each such combination is specifically contemplated and should be considered disclosed.

Reference will now be made in detail to specific aspects of the disclosed materials, compounds, compositions, articles, and methods, examples of which are illustrated in the accompanying Examples and Figures.

Described herein are nanofibers and nanofilms composed of metal oxides, polymers, or a combination thereof. Each component used to prepare the nanofiber and nanofilm is discussed in detail below. Methods for preparing and using the nanofibers and nanofilms are also outlined below.

### I. Components for Nanofiber and Nanofilm Formation

### a. Metal Oxide and Metal Oxide Precursor

The term "metal oxide" as used herein is defined as any compound containing at least one M-O-M linkage. "M" is a transition metal. It is contemplated that the metal oxide can exist only of M-O-M linkages or, in the alternative, some of the M-O-M linkages can be converted to other groups. Depending upon the selection of the organic polymer, it is possible to convert some of the M-O-M linkages to reactive functional groups such that the metal oxide can form covalent or non-covalent bonds (e.g., electrostatic, dipole-dipole, hydrogen bonding) with the organic polymer. For example, the M-O-M linkage can be reacted with acid to produce the corresponding metal hydroxide M-OH, where the hydroxyl group can interact with the organic polymer. The selection of the metal oxide can vary depending upon a number of factors including, but not limited to, compatibility with the organic polymer, compatibility with target cells, and the processability of the metal oxide.

In one aspect, the metal oxide is a transition metal oxide. Examples of transition metal oxides include, but are not limited to, niobium oxide, tantalum oxide, titanium oxide, tungsten oxide, zirconium oxide, molybdenum oxide, vanadium pentoxide, a nickel oxide, an iron oxide, a lead oxide, a germanium oxide, a manganese oxide, a cobalt oxide, a tin oxide, or a combination thereof. It is also contemplated that the metal oxide can be mixed metal oxides (*e.g.,* NiFe₂O₄). In another aspect, the metal oxide is an oxide of aluminum.

The term "metal oxide" also includes silicon compounds. Examples of silicon compound include, but are not limited to, silica, silicone, and silsesquioxanes. In one aspect, the silicon compound comprises silica. In certain aspects, the silica is amorphous.

In one aspect, the silicon compound comprises a silicone compound. Silicones, also known as polyorganosiloxanes, are synthetic polymers with a linear, repeating silicon-oxygen backbone with two organic groups bonded to each silicon atom in the chain. The organic groups prevent the formation of the three-dimensional network found in silica and can modify the physical and chemical properties of the polymer. Certain organic groups can be used to link two or more of these silicon-oxygen backbones and the nature and extent of this cross-linking enables a wide variety of products to be manufactured. Silicones can be modified after nanofiber formation to produce desired properties. Properties include porosity, wettability, chemistry, nanofiber diameter, surface area and modulus, which can be controlled by experimental variables such as composition, viscosity, molecular weight, solvent and post-modification. Depending upon the groups present on the silicone compound, silicones can exist as monomers, oligomers, or polymers. In one aspect, the silicone comprises an alkyl silicone (e.g., methyl silicone) or aryl silicone (e.g., phenyl silicone). In another aspect, the silicon compound comprises a silicone-polymer such as, for example, a silicone-polyamide or a silicone-polyurethane. The silicone-polymers and methods for preparing the same disclosed in U.S. Patent No. 6,800,713, which is incorporated by reference for its teachings of silicone-polymers, can be used herein.

In another aspect, the silicon compound comprises a silsesquioxane compound. Silsesquioxanes are silicate materials having the general formula of RSiO₃, where R is an organic group bonded to silica through a Si-C bond and the oxygen atoms bond to other silicon atoms to form a three-dimensional structure. Examples of organic groups include alkyl (e.g., methyl) and aryl (e.g., phenyl) groups. The organic group can provide functional variation in many physical properties including wettability, modulus, and chemical bonding to external surfaces. POSS™ (polyoctahedral organosilisesquioxane) molecules are cage-like silsesquioxanes. In one aspect, the silsesquioxane compound comprises a hydrophobic silsesquioxane *(e.g.,* such as methyl, phenyl, ethyl and propyl silsesquioxane), a hydrophilic silsesquioxane *(e.g.,* 2-aminopropyl silane silsesquioxane), or a crosslinkable silsesquioxane (e.g., methacryloxypropyl or glycidoxypropyl silsesquioxane).

It is contemplated that a mixture of metal oxides can be used herein. For example, any combination of silica, silicone, silsesquioxane compounds can be used as the metal oxide component.

Metal oxide precursors can be used to produce nanofibers and nanofilms. The metal oxide precursor is any compound that can be readily converted to a metal oxide as defined herein. In one aspect, the metal oxide precursor comprises a metal salt, a metal alkoxide, a metal hydroxide, a metal ester, a metal nitride, a metal carbide, a metal halide, a metal sulfide, a metal selenide, a metal phosphate, a metal sulfate, a metal carbonate, metal nitrate, a metal nitrite, a silsesquioxane, a silicone, silica, or a combination thereof. In another aspect, the metal oxide precursor comprises a niobium compound, a tantalum compound, a titanium compound, an aluminum compound, a silicon compound, a cerium compound, a calcium compound, a cadmium compound, an erbium compound, a selenium compound, a tellurium compound, a gallium compound, an arsenic compound, a germanium compound, a zinc compound, a tin compound, an indium compound, a ruthenium compound, a rhenium compound, a nickel compound, a tungsten compound, a molybdenum compound, a magnesium compound, or any combination thereof.

### b. Organic Polymer

The selection of the organic polymer can vary depending upon the metal oxide used and the desired properties of the resultant nanofiber or nanofilm. In certain aspects, the organic polymer can prevent shrinkage of the nanofiber. For example, with SiO₂, a silica nanofiber alone can shrink in the absence of the organic polymer. Here, the polymer provides structural integrity. Although not necessarily required, the organic polymer is generally a solid at room temperature. The selection of the organic polymer will also vary depending upon the solubility of the metal oxide or metal oxide precursor. As can be expected, the solubility of metal oxides can vary; however, one of ordinary skill in the art can select organic polymers that are compatible with the solvents used to dissolve the metal oxide. Thus, it is possible to use water-soluble and water-insoluble polymers.

Other factors to consider with respect to the organic polymer is the molecular weight of the polymer. A specific molecular weight polymer can be used to produce a specific nanofiber diameter. For example, 0.35 x 10⁶ MW polystyrene can be used to produce 2 to 5 µm nanofibers, 1 x 10⁶ MW polystyrene can be used to produce 0.8 µm to 2 µm diameter nanofibers, and 2 x 10⁶ MW polystyrene can be used to produce 300 nm diameter nanofibers. The molecular weight of the polymer can also influence the degree of solubility a polymer has in a given solvent system, the solution viscosity, and surface tension. The solution viscosity is an important factor with respect to nanofiber formation, in that if the solution viscosity is too great, it will not result in the formation of a random arrangement of nanofibers. Conversely, if a solution of metal oxide and organic polymer is not sufficiently viscous, "beaded" nanofibers or droplets will form.

Alternatively, one or more polymer precursors can be used to produce the polymer in *situ.* The polymer precursor is any compound capable of undergoing polymerization. Depending upon the functional groups present on the polymer precursor, the precursor can undergo polymerization via a number of different mechanisms. For example, the polymer precursor can be a polyisocyanate, which can react with a polyamine or a polyol (*e.g.,* diamine or diol, respectively) to produce a polymer *in situ.* The polymer precursor can also include other materials used to make condensation polymers including polyesters, polyamides, and polycarbonates. It is also contemplated that a polymer and a polymer precursor can be used in combination, where the two components may or may not react with one another. In one aspect, the polymer precursor can polymerize during electroprocessing (electrospinning or electrospraying) to form a polymer *in situ.*

In other aspects, the organic polymer comprises one or more functional groups capable of interacting with the metal oxide. Examples of functional groups include, but are not limited to, hydroxyl, amino, carboxyl, and the like. Depending upon the metal oxide and functional group present on the organic polymer, the interaction between the metal oxide and organic polymer can result in the formation of covalent or non-covalent bonds.

In one aspect, the organic polymer comprises polystyrene, a polyacrylate, a polyethylene, a polyimide, a polyether, a polysulfone, a polystyrenesulfonic acid, a polyethyleneimine, a polyvinyl alcohol, a polyvinylformal, a polyoxazaline, a polyvinylpyridine, a peptide, a protein, an oligonucleotide *(e.g.,* DNA or RNA), a polysaccharide, a polyamide, a polyvinylalkylether, a cycloolefinic copolymer, a polymethylmethacrylate, a polyester *(e.g.,* polyethylene terephthalate), a polymethacrylate, a phenolic compound, an epoxy compound, a urethane, a styrenic polymer (*e.g*., chlorostyrenic), maleic anhydrides, a polypropylene oxides, a polyethylene oxide, a polyolefin (*e.g*., polypropylene), a polycarbonate, a fluoropolymer, a peptides, a cellulosic polymer, a hydrogel, polylysine, a polylactic acid, polylactide-co-glycolide, an alginate, a polycaprolactone, a polyorganosilsesquioxane, an acrylamide, a polysulfonate, a polyketone, a polyacrylonitrile, a polymethylpentene, a block co-polymer, a polyvinylpyrrolidine, a polyvinyl acetate, nylon, or a combination thereof.

### II. Preparation of Nanofibers and Nanofilms

The terms "nanofiber" and "nanofilm" are defined as materials having a diameter or thickness, respectively, of 10 µm or less. In one aspect, the nanofibers can range in diameter size from 10 nm to 500 nm. In another aspect, the nanofilm has a thickness of 1 nm to 500 nm.

The nanofibers and nanofilms can be fabricated using techniques known in the art. The nanofibers and nanofilms produced herein can be composed of one or more metal oxides, one or more polymers, or a combination of one or more metal oxides and one or more polymers, which is referred to as a hybrid. In one aspect, a blend composed of a metal oxide precursor and an organic polymer can be used to produce the nanofibers or nanofilms. In one aspect, the nanofiber or nanofilm is made by the process comprising electrospinning or electrospraying, respectively, a composition comprising (1) a metal oxide, a metal oxide precursor, or a combination thereof, and (2) an organic polymer, at least one polymer precursor, or a combination thereof, to produce the nanofiber or nanofilm.

In one aspect, nanofibers can be prepared by electroprocessing. Electroprocessing includes electrospinning, electrospraying, and other methods known in the art for producing nanofibers. In embodiments of the present invention, solutions containing metal oxide, metal oxide precursor, organic polymer, polymer precursor, or a combination thereof can be electrospun to form nanofibers. Electrospinning is a technique known in the art for producing nanofibers. In another aspect, electrospray techniques known in the art can be used to produce nanofilms. Electrospraying techniques can be used to produce very thin films on a substrate surface (*e.g.,* single particles). One of ordinary skill in the art will be able to vary process parameters (*e.g.,* concentration of starting materials, voltages, etc.) to use electrospinning and electrospraying techniques to produce the fibers and films.

Prior to forming the nanofiber or nanofilm, the metal oxide, the metal oxide precursor, or a combination thereof and the organic polymer, the polymer precursor, or a combination thereof is dissolved in one or more solvents. It is also contemplated that the metal oxide, a metal oxide precursor, or a combination thereof is dissolved in one solution, and the organic polymer, the polymer precursor, or a combination thereof, is dissolved in a separate solution prior to fiber- or film-formation. The amount of metal oxide, metal oxide precursor, or a combination thereof used relative to the amount of organic polymer and/or polymer precursor can vary depending upon the solubility of the metal oxide or metal oxide precursor in the solvent system.

In one aspect, described herein is a solution for making a nanofiber comprising electrospinning a composition comprising:
(a) a metal oxide, a metal oxide precursor, or a combination of metal oxide and metal oxide precursor, and
(b) a first solvent and second solvent,
wherein the metal oxide precursor is soluble in the first solvent, the second solvent, or both the first and second solvent, wherein the first solvent and second solvent are immiscible to produce at least a two phase system.

In another aspect, described herein is a method for making a nanofiber comprising electrospinning a composition comprising:
(c) a metal oxide, a metal oxide precursor, or a combination of metal oxide and metal oxide precursor, and
(d) a first solvent and second solvent,
wherein the metal oxide precursor is soluble in the first solvent, the second solvent, or both the first and second solvent, wherein the first solvent and second solvent are immiscible to produce at least a two phase system.

In another aspect, described herein is a solution for making a nanofilm comprising electrospraying a composition comprising:
(a) a film-forming material comprising:
   (a) a metal oxide precursor;
   (b) a metal oxide and a polymer, a polymer precursor, or a combination thereof;
      or
   (c) a metal oxide precursor and a metal oxide; and
(b) a first solvent and second solvent,
wherein the film-forming material is soluble in the first solvent, the second solvent, or both the first and second solvent, wherein the first solvent and second solvent are immiscible to produce at least a two phase system.

In another aspect, described herein is a method for making a nanofilm comprising electrospraying a composition comprising:
(b) a film-forming material comprising:
   (d) a metal oxide precursor;
   (e) a metal oxide and a polymer, a polymer precursor, or a combination thereof;
      or
   (f) a metal oxide precursor and a metal oxide; and
(b) a first solvent and second solvent,
wherein the film-forming material is soluble in the first solvent, the second solvent, or both the first and second solvent, wherein the first solvent and second solvent are immiscible to produce at least a two phase system.

In a further aspect, described herein is a method for making a nanofilm comprising electrospraying a composition comprising:
(a) a film-forming material comprising:
   (i) a metal oxide precursor;
   (ii) a metal oxide and a polymer, a polymer precursor, or a combination thereof;
      or
   (iii) a metal oxide precursor and a metal oxide; and
(b) a first solvent and second solvent,
wherein the fiber-forming material is soluble in the first solvent, the second solvent, or both the first and second solvent, and the first solvent and second solvent are immiscible to produce at least a two phase system.

In these aspects, at least two solvents are selected so that at a minimum a two phase system is produced upon mixing of the solvents. The term "immiscible" includes at least two solvents that are completely insoluble with one another or at most partially soluble with one another yet forms two distinct solvent phases. The two phase system produced by the immiscible first and second solvent is responsible for the resultant morphology of the nanofiber or nanofilm after electrospinning or electrospraying. This will be demonstrated in the following example. A first solvent composed of THF contains a polymer and a second solvent composed of DMSO contains a metal oxide precursor. Upon mixing of the two solutions, a two phase system is produced because THF and DMSO are immiscible with one another. Thus, during fiber- or film formation, the THF phase with the polymer becomes "swollen" due to the presence of DMSO that is mixed with the THF during electrospinning or electrospraying. The boiling point of THF is substantially lower than that of DMSO. Thus, when THF evaporates first, relatively high amounts of DMSO with metal oxide/metal oxide precursor are dispersed throughout the polymer. Upon evaporation of the DMSO, pores are produced in the polymer and the metal oxide is left behind in the polymer.

As demonstrated in the example above, the morphology *(e.g.,* degree of porosity) of the nanofiber or nanofilm can vary depending upon the selection of starting materials, first and second solvent, and the relative amounts starting materials and first/second solvent used. Another consideration is the selection of which starting materials are soluble in a particular solvent. In one aspect, the metal oxide precursor is soluble in the first solvent and the polymer is soluble in the second solvent, wherein the first solvent has a boiling point greater than the second solvent. In another aspect, the metal oxide precursor is soluble in the first solvent and the polymer is soluble in the second solvent, wherein the first solvent has a boiling point less than the second solvent. In this aspect, smaller pore sizes can generally be produced. Thus, by selecting particular starting materials and first/second solvents, it is possible to control the morphology of the nanofiber or nanofilm that is produced. The pore size and the number of pores can also be varied using the techniques described herein. In one aspect, the methods described herein produce nanofibers and nanofilms that are highly porous throughout the entire fiber or film. The fibers also possess uniform diameters and are not beaded, which is not the case when prior art techniques are used to produce nanofibers.

The solubility of the metal oxide, the metal oxide precursor, the polymer, and the polymer precursor in the first and/or second solvent can vary depending upon the materials and solvents selected. Thus, it is contemplated that one or more of the metal oxides, the metal oxide precursors, polymers, and the polymer precursors can be soluble in the first solvent and/or the second solvent. The term "soluble" with respect to the different components ranges from completely soluble to very high solubility with only a nominal amount of insoluble material present. It is desirable in electrospinning and electrospraying techniques for the solutions to be as homogeneous as possible to avoid clogging of the needle and inconsistent spray patterns.

As an initial matter, the starting materials used to produce the nanofiber or nanofilm are selected based upon the desired end-product. Once the composition of the nanofiber or nanofilm has been identified with the desired morphology, the first solvent and second solvent are selected. The selection of the first and second solvent will be discussed in greater detail below.

In the case when a polymer is used to make the nanofiber or nanofilm, the molecular weight and viscosity of the polymer, the concentration of the polymer solution, and the diameter of the syringe needle used in electrospinning and electrospraying should be taken into consideration. Increasing the molecular weight of the polymer produces nanofibers with smaller diameters. By increasing the molecular weight of the polymer, the viscosity of the polymer increases. With an increase in polymer viscosity, lower amounts of polymer are required for nanofiber or nanofilm formation, which results the formation of thinner nanofibers. If the molecular weight is lower, the viscosity is lower and more polymer is needed for nanofiber or nanofilm formation. This results in the production of larger nanofibers or nanofilms. For example, polystyrene having a molecular weight of 350,000 produces nanofibers with larger diameters when compared to nanofibers produced from one or two million molecular weight polystyrene. In the case when nanofilms are produced by electrospraying, the polymer concentration and/or viscosity can be reduced. In one aspect, the amount of polymer and solvent that is used is sufficient to produce a viscosity of 500 cps to 5,000 cps. In another aspect, the molecular weight of the polymer is from 20,000 to 3,000,000.

In one aspect, when the nanofiber is prepared from a polymer and metal oxide precursor, the amount of polymer in the composition prior to electrospinning is from 0.1% to 50% by weight of the composition and the starting amount of metal oxide precursor in the composition prior to electrospinning is from 0.1% to 100% by weight of the composition. In another aspect, the amount of metal oxide precursor is from 1 to 40 weight %, 1 to 30 weight %, 1 to 20 weight %, 5 to 20 weight %, or 10 to 20 weight % of the composition. In another aspect, the amount of polymer is from 1 to 40 weight %, 1 to 30 weight %, 1 to 20 weight %, 5 to 20 weight %, or 10 to 20 weight % of the composition.

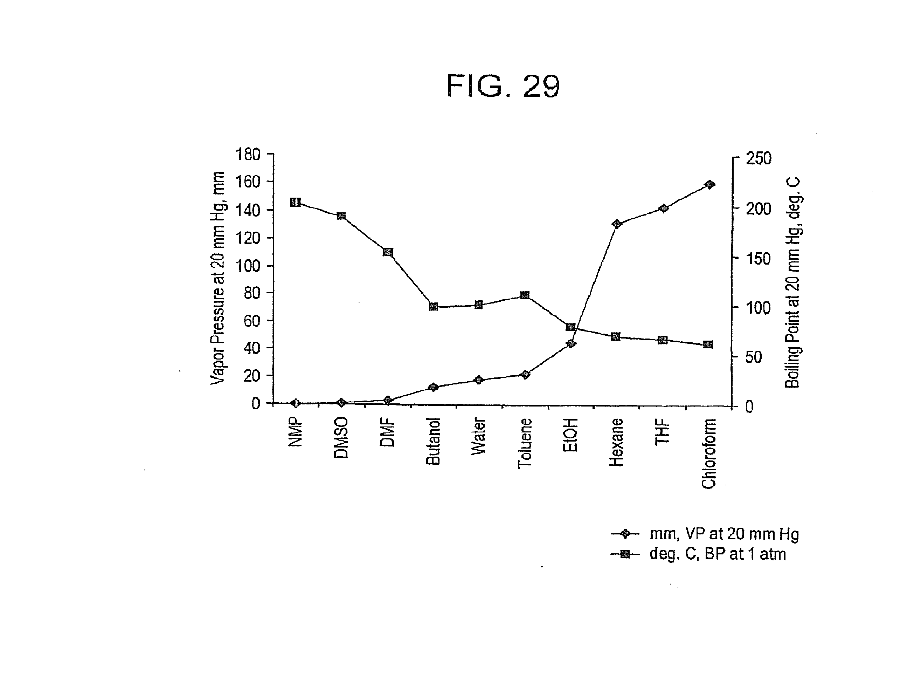

As discussed above, the first and second solvent are immiscible, which produces a two phase system. The starting materials used to prepare the nanofibers and nanofilms are an important factor with respect to selecting the first and second solvent. Another consideration that can influence the morphology of the nanofiber and nanofilm is the boiling point and vapor pressure of the first and second solvents. The solvents generally have a boiling point in the range of 50 °C to 200 °C. In one aspect, the difference in boiling points between the first and second solvent is at least 10 °C, at least 15 °C, or at least 20 °C. The vapor pressure of the solvent can between 0.1 and 170 mm at 20 °C. In another aspect, the difference in vapor pressure between the first and second solvent is at least 10 mm Hg at 20 °C, at least 20 mm Hg at 20 °C, at least 30 mm Hg at 20 °C, at least 40 mm Hg at 20 °C, or at least 50 mm Hg at 20 °C. Figure 29 is a graph showing the boiling point and vapor pressure of several solvents useful herein, which provides a useful tool in selecting the first and second solvent.

The first and second solvent can be selected from a variety of compounds such as, for example, alkanes, alkyl alcohols, carboxylic acids, or phosphonic acids. In one aspect, the first and second solvent comprises acetone, acetonitrile, carbon tetrachloride, chloroform, cyclohexane, 1,2-dichloroethane, dichloromethane, diethyl ether, dimethyl formamide, dimethyl sulfoxide, 1,4-dioxane, ethanol, methanol, ethyl acetate, heptane, hexane, methanol, methyl-tert-butyl ether, pentane, 1-propanol, 2-propanol, tetrahydrofuran, toluene, 2,2,4-trimethylpentane, water, benzene, butanol, methyl ethyl ketone, N-methyl pyrrolidine, dimethylacetamide, formic acid, acetic acid, citric acid, or any combination thereof. Provided in Table 1 is a list of solvents (first column) and description of which solvents in the first column they are immiscible or miscible with.

**Table 1**

| **Solvent** | **Solubility** |
|---|---|
| Acetone | miscible with any of the solvents listed in the column at left |
| Acetonitrile | immiscible with cyclohexane, heptane, hexane, pentane, 2,2,4-trimethyl pentane |
| carbon tetrachloride | miscible with any of the solvents listed in the column at left except water |
| chloroform | miscible with any of the solvents listed in the column at left except water |
| cyclohexane | immiscible with acetonitrile, dimethyl formamide, dimethyl sulfoxide, methanol, water |
| 1,2-dichloroethane | miscible with any of the solvents listed in the column at left except water |
| dichloromethane | miscible with any of the solvents listed in the column at left except water |
| diethylether | immiscible with dimethyl sulfoxide, water |
| dimethyl formamide | immiscible with cyclohexane, heptane, hexane, pentane, 2,2,4-trimethylpentane, water |
| dimethyl sulfoxide | immiscible with cyclohexane, heptane, hexane, pentane, 2,2,4-trimethylpentane, diethyl ether, tetrahydrofuran |
| 1,4-dioxane | miscible with any of the solvents listed in the column at left |
| ethanol | miscible with any of the solvents listed in the column at left |
| ethyl acetate | miscible with any of the solvents listed in the column at left except water |
| heptane, | immiscible with acetonitrile, dimethyl formamide, dimethyl sulfoxide, methanol, water |
| hexane | immiscible with acetonitrile, dimethyl formamide, dimethyl sulfoxide, methanol, water |
| methanol | immiscible with cyclohexane, heptane, hexane, pentane, 2,2,4-trimethylpentane |
| methyl-tert-butyl ether | miscible with any of the solvents listed in the column at left except water |
| pentane | immiscible with acetonitrile, dimethyl formamide, dimethyl sulfoxide, methanol, water |
| 1-propanol | miscible with any of the solvents listed in the column at left |
| 2-propanol | miscible with any of the solvents listed in the column at left |
| tetrahydrofuran | miscible with any of the solvents listed in the column at left except dimethyl sulfoxide |
| toluene | miscible with any of the solvents listed in the column at left except water |
| 2,2,4-tnmethylpentane | immiscible with acetonitrile, dimethyl formamide, dimethyl sulfoxide, water |
| water | immiscible with carbon tetrachloride, chloroform, cyclohexane, 1,2-dichloroethane, dichloromethane, diethyl ether, dimethyl formamide, ethyl acetate, heptane, hexane, methyl-tert-butyl ether, pentane, toluene, 2.2,4-trimethylpentane |

In certain aspects, the humidity during nanofiber or nanofilm formation can be manipulated to control the evaporation rate of the solvent(s) and/or the reaction rate of the metal oxides. Moreover, as will be discussed below, these conditions also alter the morphology of the nanofiber or nanofilm. In one aspect, the humidity is greater than 15%, greater than 30%, greater than 45%, or greater than 60%. In another aspect, the humidity is from 20 to 100%, from 30 to 100%, from 40 to 90%, or from 50 to 90%.

In certain aspects, when two immiscible solvents are used to produce a two phase system, one or more starting materials can produce co-solvents *in situ* that can drive, control, or maintain phase separation. For example, if the metal oxide precursor aluminum butoxide (Al(OBu)₃) is exposed to water *(e.g.,* water vapor present in a controlled manner), butanol is produced. Butanol may be immiscible with the first solvent or second solvent, which helps maintain phase separation. It is also contemplated that other components such as the polymer or polymer precursor can react with water vapor to produce co-solvents for phase separation. The co-solvent is different than that of the first and second solvent.

In other aspects, fiber- or film formation can be conducted in the presence of one or more solvent vapors besides water vapor. In this aspect, the organic solvent vapor can alter evaporation rates as well as phase separation. Similar to the water vapor, the organic solvent vapor can react with the components used to produce the nanofiber or nanofilm to produce co-solvents that further drive and/or control phase separation. The organic solvent vapor can be derived from any of the solvents used for the first and second solvent described above. Examples of organic solvent vapors include, but are not limited to, benzene, alcohol, DMF, DMSO, THF, or toluene. It is also contemplated that combinations of water and organic solvent vapors can be used as well. In another aspect, fiber- or film formation can be conducted in the presence of a combination of water vapor and organic solvent vapor.

The temperature during nanofiber- or nanofilm formation is also a consideration when controlling fiber or film morphology. As discussed above, the rate of evaporation of solvent during nanofiber- or film-formation can influence surface morphology. In one aspect, the temperature at which the nanofiber or nanofilm is produced is from 50° to 90 °F, 60° to 90 °F, 65° to 80 °F, or 69° to 80°F.

Other processing considerations include the pH of the solutions prior to electrospinning or electrospraying. Additionally, conditions can be adjusted so that the nanofibers or nanofilms possess a particular range of surface charge. For example, when cells, tissues, or bioactive molecules are to be immobilized on the nanofiber, it is advantageous to modify the charge to maximize immobilization efficiency. Finally, other components such as surfactants and other structure directing agents can be incorporated into the solutions prior to fiber- or film formation.

In the case when the nanofiber or nanofilm is composed of a metal oxide and polymer, the amount of metal oxide and organic polymer present in the nanofiber or nanofilm can vary. In one aspect, the amount of metal oxide is from 0.5% to 75, 0.5% to 50%, or 15 to 46% by weight of the nanofiber and the amount of organic polymer is from 25 to 99.5 % 50 to 99.5%, or 54 to 85% by weight of the nanofiber. In one aspect, when the metal oxide is silica, 40 to 59% by weight of the nanofiber is silica. In another aspect, the ratio of the length of nanofiber/diameter of nanofiber is greater than 5.

The nanofibers and nanofilms produced herein can be composed of a variety of different materials. In one aspect, the nanofiber or nanofilm comprises polystyrene and niobium oxide, tantalum oxide, titanium dioxide (titania), or any combination thereof. In another aspect, the nanofiber or nanofilm comprises polyvinyl alcohol and silica, polystyrene and alumina, polyvinylpyrrolidone (PVP) and alumina, PVP and titania, polystyrene with silica and alumina, polystyrene with alumina and titania, polystyrene and cerium oxide, PVA with cerium oxide, polyethylene oxide (PEO) with cerium oxide, or a cellulosic polymer with alumina and titania.

In one aspect, described herein is a nanofiber comprising a blend of at least one metal oxide and at least one organic polymer, wherein the metal oxide is not silica. In another aspect, described herein is a nanofiber comprising a blend of silica and at least one organic polymer, wherein the organic polymer is not polyvinyl alcohol or an ester thereof. In a further aspect, described herein is a nanofiber comprising a blend of at least one silicone compound and at least one organic polymer, wherein the organic polymer is not polylactic acid, polyglycolic acid, polylactic-co-glycolic acid, or the salt or ester thereof. In another aspect, described herein is a nanofiber comprising at least one silsesquioxane compound.

If the nanofiber or nanofilm contains a polymer, the nanofilm or nanofiber can optionally be subjected to a subsequent heat-treating step to remove the organic polymer. For example, the nanofiber or nanofilm can be calcined at elevated temperatures to produce a nanofiber or nanofilm composed only of metal oxide.

### III. Substrates for Cell/Tissue Immobilization

Described herein are substrates for immobilizing cells, tissues, and/or bioactive molecules using the nanofibers and nanofilms described herein. Upon immobilization of the cells or tissue, and/or bioactive molecule, numerous applications are contemplated. These applications will be described below.

In one aspect, described herein is a substrate for immobilizing cells or tissue comprising:
(a) a network of nanofibers or nanofilm, and
(b) a base substrate comprising a non-woven or woven porous substrate, wherein the base substrate comprises a first outer surface, wherein the network of nanofibers or nanofilm is adjacent to the first outer surface of the base substrate.

Each component of the substrates is described below.

### a. Network of Nanofibers

The nanofibers described herein can be used to produce nanofiber networks. The term "network" as used herein means a random or oriented distribution of nanofibers in space that is controlled to form an interconnecting net with spacing between nanofibers selected to promote growth and culture stability. The network has small spaces between the nanofibers comprising the network forming pores or channels in the network. The size of the pores or channels can vary depending upon the cell, tissue, or bioactive molecule to be immobilized. In one aspect, the pore size of the nanofiber network is greater than 0.2 microns. In another aspect, the pore size is less than 1 micron. In a further aspect, the pore size is from 0.2 microns to 300 microns. The network can comprise a single layer of nanofibers, a single layer formed by a continuous nanofiber, multiple layers of nanofibers, multiple layers formed by a continuous nanofiber, or mat. The network may be unwoven or net. Physical properties of the network include, but are not limited to, texture, rugosity, adhesivity, porosity, solidity, elasticity, geometry, interconnectivity, surface to volume ratio, nanofiber diameter, nanofiber solubility/insolubility, hydrophilicity/hydrophobicity, fibril density, and nanofiber orientation.

Physical properties of the nanofiber, including nanofiber size, nanofiber diameter, nanofiber spacing, matrix density, nanofiber texture and elasticity, can be important considerations for organizing the cytoskeletal networks in cells and the exposure of cell signaling motifs in extracellular matrix proteins. Physical properties of the nanofiber network that can be engineered to desired parameters include, but are not limited to, texture, rugosity, adhesivity, porosity, solidity, elasticity, geometry, interconnectivity, surface to volume ratio, nanofiber size, nanofiber diameter, nanofiber solubility/insolubility, hydrophilicity/hydrophobicity, and fibril density.

One or more of the physical properties of the nanofiber network can be varied and/or modified to create a specifically defined environment for cell/tissue immobilization. For example, porosity of the nanofiber network can be engineered to enhance diffusion of ions, metabolites, and/or bioactive molecules and/or allow cells to penetrate and permeate the nanofiber network to grow in an environment that promotes multipoint attachments between the cells and the nanofiber network. Interconnectivity of the nanofiber network can be engineered to facilitate cell-cell contacts. Elasticity of the nanofiber network can be increased or decreased by adding a bioactive molecule to the polymer solution from which the nanofibers are fabricated. It is also possible to produce nanofibers that are hollow or have a core with a sheath.

Texture and rugosity of the nanofiber network can be engineered to promote attachment of cells. For example, homogeneous or heterogeneous nanofibers can be selected to optimize growth or differentiation activity of the cells. In one aspect, the nanofiber network comprises multiple nanofibers having different diameters and/or multiple nanofibers fabricated from different polymers. In other aspects, the solubility or insolubility of the nanofibers of the nanofiber network can be engineered to control the release of bioactive molecules that can be incorporated into the nanofiber network. For example, the rate of release of bioactive molecules is determined by the rate of biodegradation or biodissolution of the nanofibers of the nanofiber network. In other aspects, the hydrophobicity and hydrophilicity of the nanofiber network can be engineered to promote specific cell spacing.

The layering of individual single layer nanofiber networks can form channels, which allow diffusion of ions, metabolites, proteins, and/or bioactive molecules as well as permit cells to penetrate the nanofiber network and grow in an environment that promotes multipoint attachments between the cells and the nanofiber network.

The network of nanofibers can produce a three dimensional environment similar to that found *in vivo.* In particular, to achieve efficient cell culturing that is comparable to *in vivo* cell growth, it is desirable that the material permit the permeation of cells through the entire material. One function of the three dimensional environment is to direct cell behavior such as migration, proliferation, differentiation, maintenance of the phenotypes and apoptosis by facilitating sensing, and responding to the environment via cell-matrix and cell-cell communications. Therefore, a material having proper porosity, large surface area, and well inter-connected pores is desirable for culturing cells. The nanofibers produced herein possess these features.

### b. Base Substrate

The term "base substrate" as used herein means any surface on which the network of nanofibers or nanofilm can be deposited. The base substrate can be any surface that offers structural support for the deposited network of nanofibers or nanofilm. In one aspect, the base substrate can comprise glass, cellulose, or plastic. In another aspect, the base substrate can be a film, a woven mat, a non-woven mat, or an article.

The base substrate can be porous or non-porous. The porosity of the base substrate can vary depending upon the application of the substrate. For example, when the substrate is used to immobilize cells, the porosity of the base substrate can be determined by cellular penetration. A cell is able to penetrate a porous substrate but is not able to penetrate a non-porous substrate. Depending upon the porosity of the nanofiber network or nanofilm, the base substrate can have pores that are greater or smaller in diameter to the pores present in the nanofiber network or nanofilm. It is contemplated that cells can penetrate and be retained by the base substrate and/or the network of nanofibers or nanofilm. The size of the pores in the base substrate can vary depending upon the cell or tissue to be immobilized. In one aspect, the pore size is greater than 0.2 microns. In another aspect, the pore size is less than 1 micron. In a further aspect, the pore size is from 0.2 microns to 300 microns.

Any of the polymers described above for producing nanofibers or nanofilms can be used to produce the base substrate. Examples of such polymers include, but are not limited to, a polyolefin, cyclic polyolefin, polyacetal, polyamide, polyester, polycarbonate, cellulose ether and ester, polyalkylene sulfide, polyarylene oxide, polyalkylene oxide, copolymers and block copolymers of alkylene oxide, polyvinylcarbazole, polysulfone, modified polysulfone polymers and mixtures thereof. Preferred materials that fall within these generic classes include polyethylene, poly(epsilon-caprolactone), a polylactide, a polyglycolide, a polylactide-co-glycolide, polypropylene, polysiloxane, poly(vinylchloride), polyvinylpyrrolidone, polyvinyl acetate, polymethylmethacrylate (and other (meth)acrylic resins), poly (meth)acrylamide, polystyrene, and copolymers thereof (including ABA type block copolymers), poly(vinylidene fluoride), poly(vinylidene chloride), polyvinyl alcohol in various degrees of hydrolysis (87% to 99.5%) in crosslinked and non-crosslinked forms. It is contemplated that the base substrate can be composed of layers of different polymers or composed of a blend of two or more polymers. Any of the polymers described above can be woven or non-woven to produce the base substrate. For example, the base substrate can be composed of Nylon nanofibers woven into a mat.

### c. Bioactive Molecules

The nanofiber network, nanofilm, and/or the base substrate can comprise one or more bioactive molecules. In one aspect, the network of nanofibers, nanofilm, or base substrate comprises one or more compounds for enhancing cell/tissue growth. In another aspect, the nanofiber, nanofilm, or base substrate further comprises a compound that promotes attachment of a cell or tissue to the nanofiber or substrate.

Bioactive molecules include human or veterinary therapeutics, nutraceuticals, vitamins, salts, electrolytes, amino acids, peptides, polypeptides, proteins, carbohydrates, lipids, polysaccharides, nucleic acids, nucleotides, polynucelotides, glycoproteins, lipoproteins, glycolipids, glycosaminoglycans, proteoglycans, growth factors, differentiation factors, hormones, neurotransmitters, pheromones, chalones, prostaglandins, immunoglobulins, monokines and other cytokines, humectants, minerals, electrically and magnetically reactive materials, light sensitive materials, anti-oxidants, molecules that may be metabolized as a source of cellular energy, antigens, and any molecules that can cause a cellular or physiological response. Any combination of molecules can be used, as well as agonists or antagonists of these molecules. Glycoaminoglycans include glycoproteins, proteoglycans, and hyaluronan. Polysaccharides include cellulose, starch, alginic acid, chytosan, or hyaluronan. Cytokines include, but are not limited to, cardiotrophin, stromal cell derived factor, macrophage derived chemokine (MDC), melanoma growth stimulatory activity (MGSA), macrophage inflammatory proteins 1 alpha (MIP-1 alpha), 2, 3 alpha, 3 beta, 4 and 5, interleukin (IL) 1, IL-2, IL-3, IL-4, IL-5, IL-6, IL-7, IL-8, IL-9, IL-10, IL-11, IL-12, IL-13, TNF-alpha, and TNF-beta. Immunoglobulins useful in the present invention include, but are not limited to, IgG, IgA, IgM, IgD, IgE, and mixtures thereof. Amino acids, peptides, polypeptides, and proteins can include any type of such molecules of any size and complexity as well as combinations of such molecules. Examples include, but are not limited to, structural proteins, enzymes, and peptide hormones.

The term bioactive molecule also includes fibrous proteins, adhesion proteins, adhesive compounds, deadhesive compounds, and targeting compounds. Fibrous proteins include collagen and elastin. Adhesion/deadhesion compounds include fibronectin, laminin, thrombospondin and tenascin C. Adhesive proteins include actin, fibrin, fibrinogen, fibronectin, vitronectin, laminin, cadherins, selectins, intracellular adhesion molecules 1, 2, and 3, and cell-matrix adhesion receptors including but not limited to integrins such as α₅β₁, α₆β_{1,} α₇β₁, α₄β₂, α₂β₃, and α₆β₄.

The term bioactive molecule also includes leptin, leukemia inhibitory factor (LIF), RGD peptide, tumor necrosis factor alpha and beta, endostatin, angiostatin, thrombospondin, osteogenic protein-1, bone morphogenic proteins 2 and 7, osteonectin, somatomedin-like peptide, osteocalcin, interferon alpha, interferon alpha A, interferon beta, interferon gamma, interferon 1 alpha, and interleukins 2, 3, 4, 5 6, 7, 8, 9, 10, 11, 12, 13, 15, 16, 17 and 18.

The term "growth factor" as used herein means a bioactive molecule that promotes the proliferation of a cell or tissue. Growth factors useful in the present invention include, but are not limited to, transforming growth factor-alpha. (TGF-alpha), transforming growth factor-beta. (TGF-beta), platelet-derived growth factors including the AA, AB and BB isoforms (PDGF), fibroblast growth factors (FGF), including FGF acidic isoforms 1 and 2, FGF basic form 2, and FGF 4, 8, 9 and 10, nerve growth factors (NGF) including NGF 2.5s, NGF 7.0s and beta NGF and neurotrophins, brain derived neurotrophic factor, cartilage derived factor, bone growth factors (BGF), basic fibroblast growth factor, insulin-like growth factor (IGF), vascular endothelial growth factor (VEGF), EG-VEGF, VEGF-related protein, Bv8, VEGF-E, granulocyte colony stimulating factor (G-CSF), insulin like growth factor (IGF) I and II, hepatocyte growth factor, glial neurotrophic growth factor (GDNF), stem cell factor (SCF), keratinocyte growth factor (KGF), transforming growth factors (TGF), including TGFs alpha, beta, beta1, beta2, and beta3, skeletal growth factor, bone matrix derived growth factors, and bone derived growth factors and mixtures thereof. Some growth factors can also promote differentiation of a cell or tissue. TGF, for example, can promote growth and/or differentiation of a cell or tissue. Some preferred growth factors include VEGF, NGFs, PDGF-AA, PDGF-BB, PDGF-AB, FGFb, FGFa, and BGF.

The term "differentiation factor" as used herein means a bioactive molecule that promotes the differentiation of cells or tissues. The term includes, but is not limited to, neurotrophin, colony stimulating factor (CSF), or transforming growth factor. CSF includes granulocyte-CSF, macrophage-CSF, granulocyte-macrophage-CSF, erythropoietin, and IL-3. Some differentiation factors may also promote the growth of a cell or tissue. TGF and IL-3, for example, can promote differentiation and/or growth of cells.

The term "adhesive compound" as used herein means a bioactive molecule that promotes attachment of a cell or tissue to a nanofiber surface comprising the adhesive compound. Examples of adhesive compounds include, but are not limited to, fibronectin, vitronectin, and laminin.

The term "deadhesive compound" as used herein means a bioactive molecule that promotes the detachment of a cell or tissue from a nanofiber comprising the deadhesive compound. Examples of deadhesive compounds include, but are not limited to, thrombospondin and tenascin C.

The term "targeting compound" as used herein means a bioactive molecule that functions as a signaling molecule inducing recruitment and/or attachment of cells or tissues to a nanofiber comprising the targeting compound. Examples of targeting compounds and their cognate receptors include attachment peptides including RGD peptide derived from fibronectin and integrins, growth factors including EGF and EGF receptor, and hormones including insulin and insulin receptor.

The incorporation of the bioactive molecule into the nanofiber network or base substrate can be accomplished by a variety of techniques. For example, during the formation of the nanofiber or nanofilm, one or more bioactive molecules can be in the first and/or second solution so that during electrospinning or electrospraying, respectively, the bioactive molecule is incorporated throughout the fiber or film. In another aspect, the bioactive molecule can be applied to the surface of the nanofiber, nanofilm or base substrate using techniques known in the art *(e.g.,* spraying, dipping, etc.). Depending upon the selection of the bioactive molecule and the materials used to produce the nanofiber, nanofilm, or base sub strate.

The bioactive molecules can be incorporated into the nanofiber network, nanofilm, or the base substrate during fabrication or can be attached to a surface of the network, nanofilm, or substrate via a functional group so that the bioactive molecule is covalently or non-covalently attached to the nanofiber network, nanofilm, or the base substrate. In certain aspects, one or more functional groups can be incorporated on the outside surface of the nanofibers, nanofilm, or base substrate. These functionalized surfaces can bind a peptide, polypeptide, lipid, carbohydrate, polysaccharide, amino acid, nucleotide, nucleic acid, polynucleotide, or other bioactive molecules to the surface of the nanofiber or base substrate. In one aspect, the functional groups are deposited on the outside surface of the nanofiber or base substrate by plasma deposition. Plasma deposition creates local plasmas at the surface of the nanofiber, nanofilm, or base substrate. The treated surface is then reacted with gaseous molecules, such as for example, allylamine and/or allyl alcohol, in a reaction chamber. In another aspect, the functional groups are introduced onto the surface of the nanofibers or nanofilm during the electrospinning or electrospraying process, respectively. For example, dodecyl amine, dodecyl aldehyde, dodecyl thiol, or dodecyl alcohol can be added to the polymer solution. The polymer solution is then electrospun into nanofibers in which a portion of the added amines, aldehydes, sulphydryl, or alcohol moieties, respectively, are exposed on the outside surface of the nanofibers.

### d. Preparation of Substrates for Cell/Tissue Immobilization

The nanofiber network or nanofilm can be deposited on the base substrate using techniques known in the art. In one aspect, the nanofiber network can be produced and deposited on the base substrate by charging techniques such as, for example, corona charging and tribocharging. Alternatively, the nanofiber network can be electrospun onto the base substrate such that the nanofiber network is adjacent to the base substrate. Similarly, the nanofilm can be electrosprayed on the on the base substrate. In other aspects, a preformed nanofiber network can be attached to the base substrate with the use of an adhesive.

The term "adjacent" as used herein includes the intimate contact between the nanofiber network or nanofilm and the surface of the base substrate. The term "adjacent" also includes one or more layers interposed between the nanofiber network or nanofilm and the base substrate. For example, an adhesion protein can be deposited on the outer surface of the base substrate prior to depositing the nanofiber network on the base substrate. In one aspect, cells or tissue are not interposed between the nanofiber network and the base substrate. As described above, electrospinning can be used to produce nanofibers with different properties and orientations as desired. In general, although not prohibited, the other exposed surface of the base substrate does not have any components adjacent to the other exposed surface. Upon deposition of the nanofibers on the base substrate, the nanofibers are evenly distributed on the base substrate at a uniform thickness.

It is also contemplated that two or more nanofiber networks or nanofilms can be layered on the base substrate. For example, different nano- and/or micro-environments that promote cellular activity of a particular cell or tissue can be constructed by layering different nanofiber networks that have selected physical and/or chemical properties. The physical and/or chemical properties can be engineered into the individual nanofiber networks as described above. The layering of individual nanofiber networks can form channels that allow diffusion of ions, metabolites, proteins, and/or bioactive molecules as well as permit cells to penetrate the substrate and grow in an environment that promotes multipoint attachments between the cells and the nanofiber network or nanofilm.

### IV. Kits

In another aspect, described herein is a kit comprising a network of nanofibers or a nanofilm and a base substrate. Any of the nanofiber networks, nanofilms, and base substrates described above can be used herein. In one aspect, one or more pre-manufactured nanofiber networks can be individually wrapped and sterilized. After removal from the packaging, one or more nanofiber networks can be assembled manually or mechanically on the base substrate. In the case of multiple nanofiber networks, each nanofiber network can be applied to the base substrate layer by layer to form a multi-layered assembly. The base substrate can be an article such that it is shaped to receive the nanofiber network.

### V. Applications

The substrates described herein are used to immobilize cells or tissues. The term "immobilization" as used herein is the ability of the substrate to retain the cell or tissue. Immobilization can range from completely retaining the cell or tissue such that the cell or tissue is locked in position within the nanofiber network or base substrate to a situation where the cell or tissue can freely permeate the nanofiber network or base substrate. The incorporation of bioactive molecules into the nanofiber network, nanofilm, or base substrate can determine the degree of immobilization of the cell or tissue on the substrate.

The substrates described herein can be used in a number of applications, which are described below. It is contemplated that the substrates can be used in many known applications employing nanofibers including, but not limited to, filter applications, pharmaceutical applications, cell culture, tissue culture, and tissue engineering. It is contemplated one or more cell types can be deposited on the substrate. The cells can be deposited on the substrate using techniques known in the art.

In one aspect, described herein is a method for growing a plurality of cells, comprising (a) depositing a parent set of cells on a substrate described herein, and (b) culturing the substrate with the deposited cells to promote the growth of the cells.

In another aspect, described herein is a method for differentiating cells, comprising (a) depositing a parent set of cells on a substrate described herein, and (b) culturing the assembly to promote differentiation of the cells.

Many types of cells can be immobilized on the substrate including, but not limited to, stem cells, committed stem cells, differentiated cells, and tumor cells. Examples of stem cells include, but are not limited to, embryonic stem cells, bone marrow stem cells and umbilical cord stem cells. Other examples of cells used in various embodiments include, but are not limited to, osteoblasts, myoblasts, neuroblasts, fibroblasts, glioblasts, germ cells, hepatocytes, chondrocytes, keratinocytes, smooth muscle cells, cardiac muscle cells, connective tissue cells, glial cells, epithelial cells, endothelial cells, hormone-secreting cells, cells of the immune system, and neurons.

Cells useful herein can be cultured *in vitro,* derived from a natural source, genetically engineered, or produced by any other means. Any natural source of prokaryotic or eukaryotic cells can be used.

Atypical or abnormal cells such as tumor cells can also be used herein. Tumor cells cultured on substrates described herein can provide more accurate representations of the native tumor environment in the body for the assessment of drug treatments. Growth of tumor cells on the substrates described herein can facilitate characterization of biochemical pathways and activities of the tumor, including gene expression, receptor expression, and polypeptide production, in an in vivo-like environment allowing for the development of drugs that specifically target the tumor.

Cells that have been genetically engineered can also be used herein. The engineering involves programming the cell to express one or more genes, repressing the expression of one or more genes, or both. Genetic engineering can involve, for example, adding or removing genetic material to or from a cell, altering existing genetic material, or both. Embodiments in which cells are transfected or otherwise engineered to express a gene can use transiently or permanently transfected genes, or both. Gene sequences may be full or partial length, cloned or naturally occurring.

By varying and/or modifying selected physical and/or chemical properties of the substrate, the substrate can be engineered to promote cellular growth of a particular cell or tissue. The physical properties and/or characteristics of the substrate including, but not limited to, texture, rugosity, adhesivity, porosity, elasticity, solidity, geometry, and fibril density can be varied and/or modified to promote a desired cellular activity, including growth and/or differentiation. Specific nano- and/or micro-environments can be engineered within the substrate. For example, the porosity and fibril density of the substrate can be varied and/or modified to allow a cell to penetrate the substrate and grow in a three dimensional environment. Any of the bioactive molecules described herein can be engineered into the substrate either isotropically or as gradients to promote desired cellular activity, including cell adhesion, growth, and/or differentiation. The physical and/or chemical properties of the substrate, including growth and differentiation factors, on which such cells are grown can be engineered to mimic the native *in vivo* nano- or micro-environments.

With designed patterns, the spatial organization of the cells in two and three dimensions can be obtained. By creating specific patterns of surface chemistry, cell behavior can be confined within physical or chemical ultrastructures, which can be used to control cellular activity such as cell growth and/or proliferation.

In another aspect, described herein is method for growing tissue, comprising (a) depositing a parent set of cells that are a precursor to the tissue on a substrate described herein, and (b) culturing the substrate with the deposited cells to promote the growth of the tissue. It is also contemplated that viable cells can be deposited on the substrates described herein and cultured under conditions that promote tissue growth. Tissue grown (i.e., engineering) from any of the cells described above is contemplated with the substrates described herein. The substrates described herein can support many different kinds of precursor cells, and the substrates can guide the development of new tissue. The production of tissues has numerous applications in wound healing. Depending upon the selection of materials used to produce the nanofibers and base substrate, tissue growth can be performed *in vivo* or *ex vivo.*

In certain instances, it is desirable to remove the cells or tissue from the substrate. For example, it would be desirable to harvest stem cells that have been growing on the substrates described herein. Invasive techniques known in the art for removing cells include, but are not limited to, mechanical scraping, sonication, chemical/enzymatic treatment, or a combination thereof. Other techniques involve adjusting the pH or temperature or the addition of ions to release attached cells.

In another aspect, described herein are methods for determining an interaction between a known cell line and a drug, comprising (a) depositing the known cell line on a substrate described herein; (b) contacting the deposited cells with the drug; and (c) identifying a response produced by the deposited cells upon contact with the drug.

With a known cell line immobilized on the substrates described herein, it is possible to screen the activity of several drugs when the drug interacts with the immobilized cells. Depending upon the cells and drugs to be tested, the cell-drug interaction can be detected and measured using a variety of techniques. For example, the cell may metabolize the drug to produce metabolites that can be readily detected. Alternatively, the drug can induce the cells to produce proteins or other biomolecules. The substrates described herein provide an environment for the cells to more closely mimic the *in vivo* nature of the cells in an *ex vivo* environment. The substrates can be used in high throughput applications for analyzing drug/cell interactions. High throughput applications utilize multiwell tissue culture chambers with densities up to about 1536 wells per plate. Thus, increasing the population of cells per well would serve to increase the measured signals.

In another aspect, described herein are methods for separating a compound present in a solution, comprising (a) contacting the solution with a substrate described herein, wherein the compound is immobilized on the substrate; and (b) removing the immobilized compound from the substrate. The nanofiber network and/or base substrate can be modified to immobilize any of the bioactive molecules described above in solution. In general, a solution composed of one or more bioactive molecules is contacted with the substrate, at which time the bioactive molecule is immobilized on the substrate. The bound bioactive molecule can then be released from the substrate with a solvent. The substrate can be modified as described above so that the substrate forms a covalent or non-covalent (e.g., ionic, electrostatic dipole-dipole, Van Der Waals interactions) bond with the bioactive molecule. In another aspect, cells can be purified. For example, by measuring the electric properties of a single individual cell immobilized on the substrate, it is possible to sort/purify a population of cells by their different intrinsic electric properties. This application can be of particular interest in stem cells, where it is desirable to harvest large quantities of pure stem cells.

In other aspects, the nanofibers and nanofilms described herein can be used as supports for a variety of different materials. Such materials include a catalyst, a metallic or organic conductive material, a magnetic material, a piezoelectric material, a ferroelectric material, a dielectric material, a radioactive material, a phosphorescent material, a dye, a surfactant, or a rare earth metal. The amounts of these components will vary depending upon the intended end-use of the, which can be readily determined by one of ordinary skill in the art.

### EXAMPLES

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations of the present invention which are apparent to one skilled in the art.

Efforts have been made to ensure accuracy with respect to numbers *(e.g.,* amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, *e.g.,* component concentrations, temperatures, pressures and other reaction ranges and conditions that can be used to optimize the product purity and yield obtained from the described process. Only reasonable and routine experimentation will be required to optimize such process conditions.

### EXAMPLE 1-Niobium, Tantalum, or Titanium/Polystyrene Nanofibers Sample Preparation

Samples were prepared from either pure polystyrene (PS) or polystyrene mixed with either niobium ethoxide (Catalog # 339202 Sigma Aldrich) or tantalum butoxide (Catalog # 383333 Sigma Aldrich). In the case of polystyrene alone, PS was dissolved in tetrahydrofuran (THF) in a concentration range of 18-20%. Molecular weight ranges of polystyrene from 350,000 to 2,000,000 were used to target different nanofiber diameters. For the case of tantalum butoxide or niobium ethoxide polystyrene blends, polystyrene was first dissolved in THF and the inorganic oxide was dissolved in glacial acetic acid with or without DMSO. Tantalum and niobium were in a concentration range of 15 to 49%.

### Electrospinning Process

Nanofibers were spun either on glass cover slips, silver coated glass, or polyvinylacetate cover slips. Voltage was between 5 and 9 kV and the nanofiber to substrate distance varied between 5 and 15 cm. The relative humidity was controlled and could vary between 40 and 70% and had a significant impact on nanofiber surface texture with increased humidity resulting in increased surface area. Temperature of the electrospinning process varied from 68 to 75 °F. The number of cycles at 2 rpm varied from 20 to 250 to allow for variations in mat density.

### Cell Lines

Cell lines used to study attachment and proliferation included MRC5, HEPG2 and human mesenchymal stem cells (HMSCs). The cell line used to study attachment, proliferation and function was the HEPG2 liver cell line (ATCC # HB-8065). MRC5 cells were likewise obtained from ATCC (# CCL-171) while human mesenchymal stem cells were obtained from Cambrex (#PT2501). All cell culture took place in Isocove's Modified Dulbecco's Medium (IMDM) + 10% Fetal Bovine Serum (FBS) except for the HMSCs which were grown in media offered through Cambrex. IMDM was catalog # 12440 -053 from Gibco while fetal bovine serum was from Hyclone. When adding cells to substrates, 1% Penicillin / Streptomycin (Catalog # 15140-122) from Invitrogen was added to the mixture to prevent contamination.

Substrates were always washed with 70% ethanol and subsequently with IMDM + 10% FBS + 1% Penicillin /Streptomycin prior to addition of cells. The MTT cell proliferation assay (ATCC, Catalog # 30-101OK) or Cell TITER 96® AQ One Solution Cell Proliferation Assay (Promega, Catalog # G3580) were used to assess for cell attachment and proliferation. Hepatocyte protein production was measured by using the BCA Assay from Pierce Biotechnologies (Product # 23227). Albumin production assays from Biomeda (Catalog # EU1057) or AssayPro (Catalog # EA3201-1) were utilized to assess for hepatocyte albumin production.

### Results

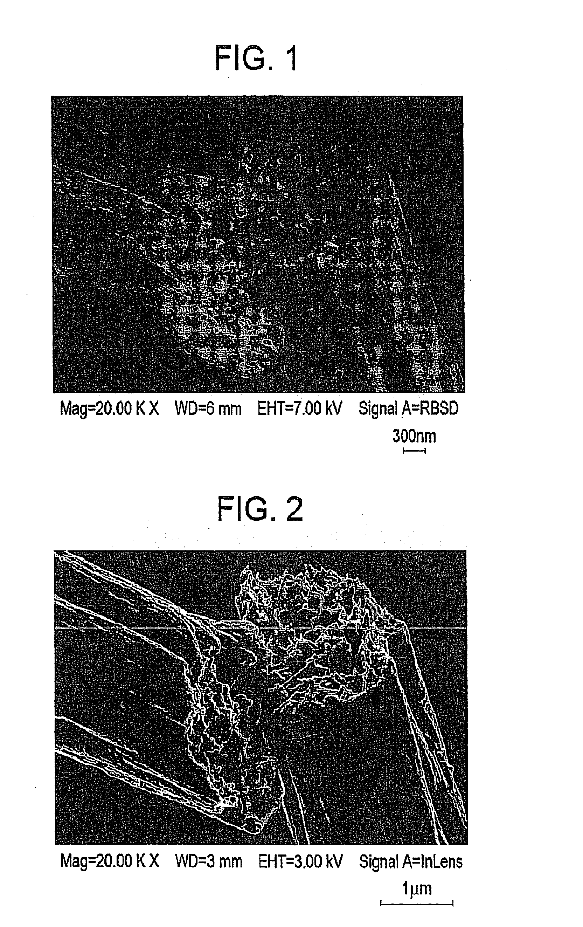

Figure 1 shows the texture of nanofibers as revealed by SEM. Niobia/ Polystyrene without DMSO shows a smoother texture compared to niobia/polystyrene with DMSO or tantala/polystyrene with DMSO.

Figure 2 shows SEMs of nanofiber texture inside the nanofiber (top) as well as SEM backscattered images (bottom), where white points are indicative of the heavy metal. Tantala / polystyrene nanofibers were spun with DMSO. Inhomogeneous mixing of the organic/inorganic that occurs with addition of DMSO may provide inorganic reactive centers with which to modify the nanofiber and enhance cell culture. White arrows indicate regions of the inorganic phase.

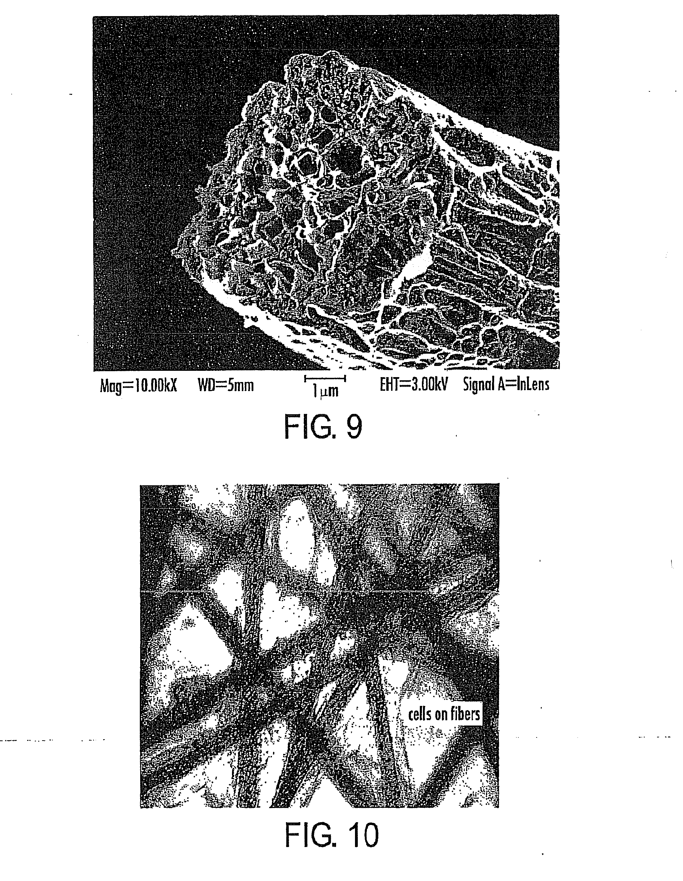

Figure 9 shows the texture of titania/polystyrene nanofibers as revealed by SEM. The nanofiber was prepared with the use of THF/DMSO, and the nanofiber is porous on the surface as well as throughout the entire nanofiber.

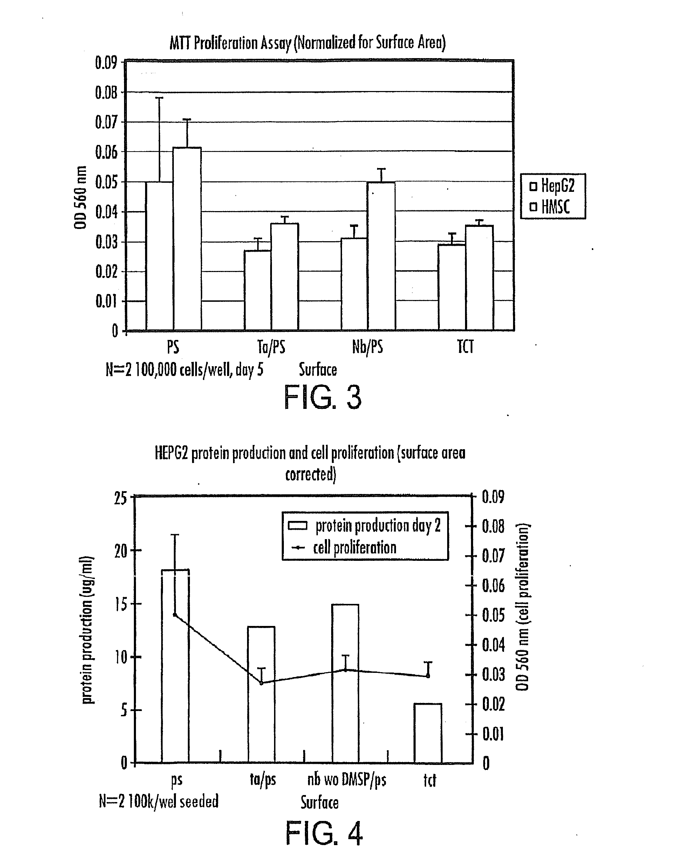

Figure 3 shows proliferation was greater than or equal to tissue culture treated polystyrene (TCT) for electrospun substrates for both the MRC5 cell line and HMSCs. In this case, two substrates of each substrate type were tested. 100,000 cells were seeded on day 1. Proliferation was tested by using the ATCC MTT assay on day 5. In Figure 4, HEPG2 cell proliferation and total protein production were compared to TCT for the different electrospun substrates. The total protein production was determined by the BCA assay and cell proliferation was measured at day 2.

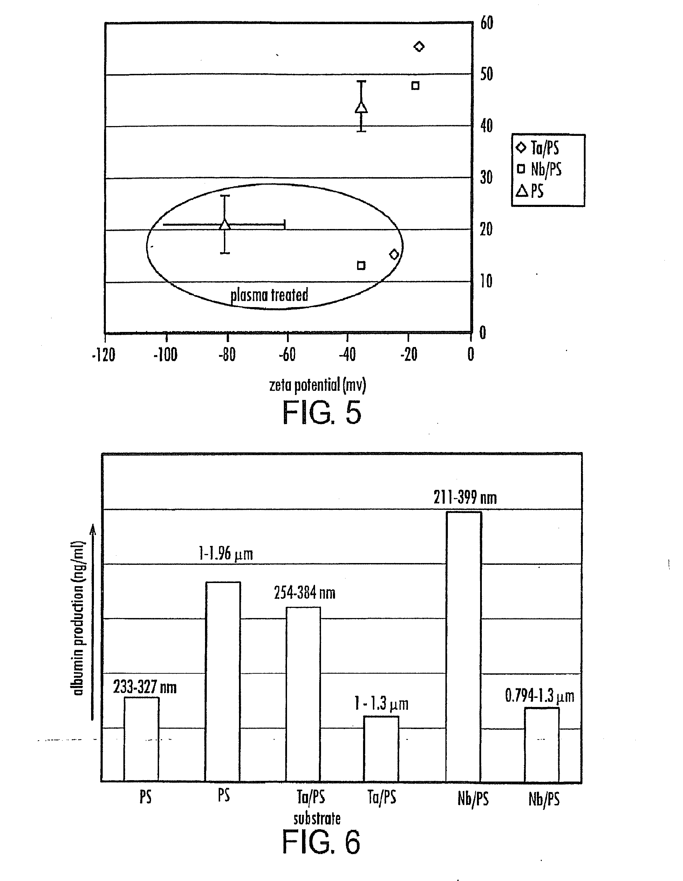

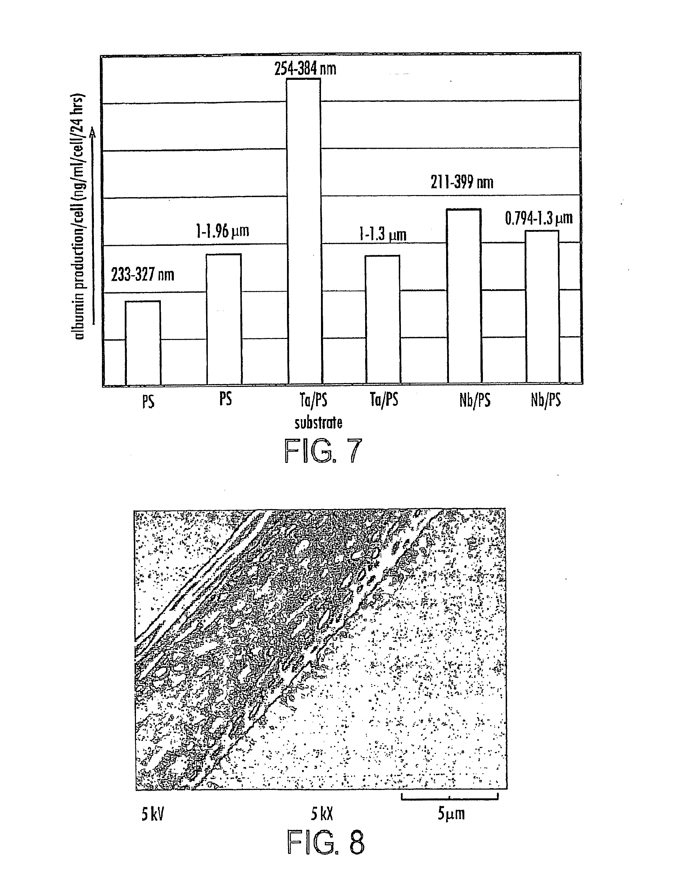

Figure 5 shows protein production as a function of surface charge as measured by zeta potential at a pH 7.0 in 1mM KCl. Figure 6 shows albumin production as a function of substrate. In this case, electrospun substrates were placed in a 6 well plate and 500,000 HEPG2 cells were seeded on day 1. On day 3, media was replaced. On day 5, the substrates were removed, placed in fresh wells and covered with DPBS plus Ca⁺² plus Mg⁺². On day 6, media was removed and assessed for albumin. If normalizing for cell count, a Promega CellTiter Assay was done at the same time as assaying for albumin. When using TCT, Matrigel™ or other substrates that were already offered in a 6 well format as a control, the same protocol was followed, except substrates were removed and put in a fresh TCT plate to determine the amount of cells on the electrospun substrate only. Comparison with a 6 well control was done by normalizing with respect to the different nominal surface areas. Figure 7 shows albumin production as a function of substrate normalized for cell count determined at the same time as albumin was assayed.

### EXAMPLE 2-Titania/Polystyrene Nanofibers

A cell culture surface or scaffold consisting of titania/polystyrene nanofibers of submicron-micron diameter was deposited by electrospinning oxide/polymer hybrid nanofibers onto a polymer or glass surface. These nanofibers have a unique surface morphology, internal porosity and chemical composition, which make them an excellent substrate for adhesion and growth of animal cells. The nanofiber structure and diameter approximates that of the extracellular matrix, which is deposited as a basement surface by animal cells during culture. The biomimetic topography of this surface is such that it provides an advantageous three-dimensional substrate for the growth of animal cells in culture, resulting in significantly improved cell yield over the standard non-fibrous cell culture surfaces. Figures 8 and 9 show the surface morphology and features of titania/polystyrene nanofibers.

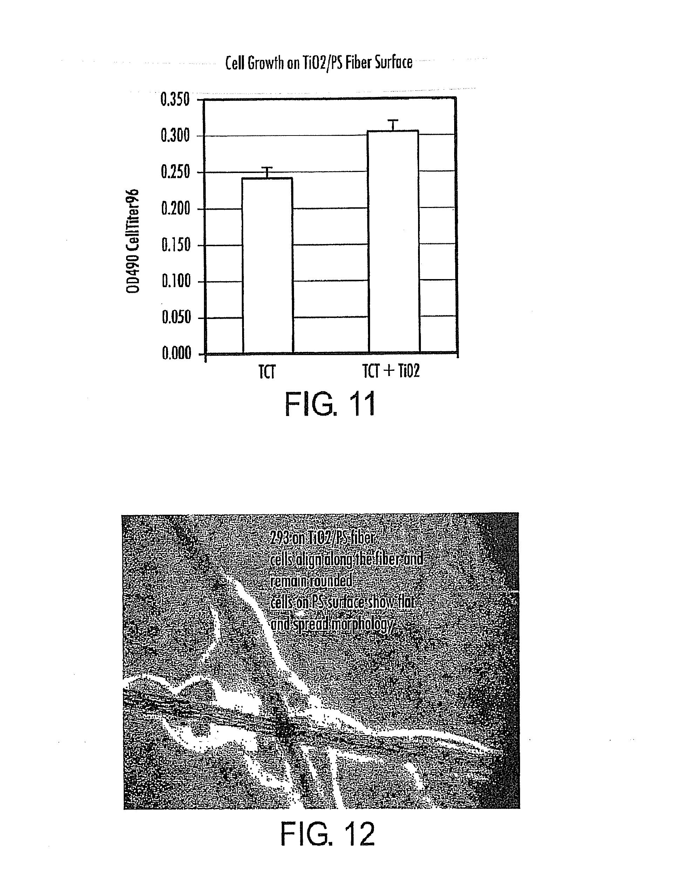

HEK293 cells attached and grew on these hybrid nanofiber surfaces (Figure 10). Significantly more cell growth was seen on the hybrid nanofiber surface than on the tissue culture treated polystyrene control surface after 3-5 days under standard cell culture conditions (Figure 11). Additionally, these cells exhibited a rounded morphology when grown on the titania/polystyrene hybrid nanofibers, and aligned along the nanofiber length (Figure 12). Cells grew preferentially between, among and along the nanofibers, resulting in a three-dimensional cell mass, rather than in the flattened monolayer typically found on tissue culture treated (oxidized) polystyrene. This cell growth morphology is desired because it more closely resembles the in-vivo cell morphology.

Preliminary analysis of these electro-spun hybrid nanofibers by an electron microprobe indicated a uniform distribution of titania within the nanofiber. BET measurements show a higher surface area value (14 m²/gram) than smooth dense nanofibers. This result agrees well with the observed surface features and internal porosity shown in Figures 8 and 9. The typical nanofiber diameters are on the order of 0.5 to 5 micrometers with the most common nanofibers being 2 to 3 micrometers in diameter. The nanofiber surface has axially oriented grooves with 50% or more of the nanofiber surface being covered by a porous and at times web-like coating. This coating facilitates the adherence of the nanofibers to each other and the substrate surface, resulting in a stable culture substrate or scaffold.

### EXAMPLE 3-Silica/Polystyrene Nanofibers and Silicone/Polystyrene Nanofibers Materials

Silica nanofibers were formed using known sol-gel technology by varying the catalyst, pH, and water/alkoxide ratio. A polymer was introduced to this composition that would swell in water and hence reduce shrinkage of the mat, and also act as a binder for the inorganic component. Several compositions were studied and typically these consist of TEOS/water (+/- DMSO)/acid and polymer. Solution details are below.

Step 1: TEOS (5.5 g), water (5.5 g) and 2 drops of H₃PO₄ (42.5% strength)(0.08 g) were mixed with vigorous stirring for 15 min or until clear. Then heated solution for 1h at 60 °C, and then keep solution in an ice bath to slow down further reaction.

Step 2: Mixed the following components as a separate solution, TEOS mixture from step 1 (2 g), H₂O (0.53 g), DMSO (0.25 g) and 8% polyvinyl alcohol (PVA:MW 89-98K, 6.25g). The solution was heated at 60 °C for one hour, cooled to room temperature, and aged for 3 days before electrospinning. Resultant silica in the final mat composition was varied between 40-59% by varying the polymer content. Solutions were also made without DMSO.

Since PVA is partially water-soluble, the electrospun mats are post-modified using a chemical cross-linker such as glutaraldehyde. Porous mats consisting of 200-400 nm diameter nanofibers of silica/ PVA hybrids were prepared. Different silane (amine, TEOS) coated glass coverslips were used as the electrospinning substrate to assist in the adhesion of nanofibers and hence increase the density of the mats.

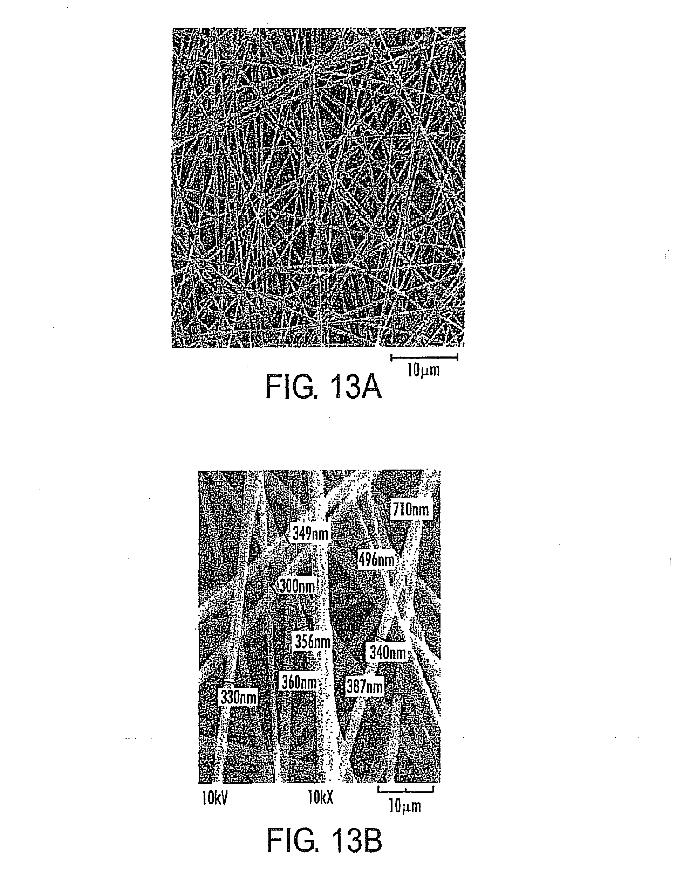

Figure 13 shows SEMs of these nanofibers depicting their unique surface morphology and typical nanofiber diameters. The silica/PVA hybrid nanofiber diameters (100-400 nm) approximate that of the fibrillar basement membrane, a structurally compact form of the extracellular matrix (ECM). Silica is an essential nutrient and PVA is known as a synthetic ECM like protein and hence this system could be a good biomimetic for cell culture.

Silicone/organic blend have been formed by electrospinning a silicone containing blockcoplymer/PS hybrid. There are many commercially available silicones with variable viscosity, molecular weight and composition. Low glass transition temperature (Tg) of most of the commercially available silicones makes it impossible to be electrospun alone but these could be electrospun as a copolymer blend. Several compositions were studied.
#1 Mixture of PS (MW 350, 000, 6 g of PS in 30 ml of THF and 0.5 ml acetic acid, ∼20% in THF) and copolymer (35-45% polydimethylsiloxane)phenylenediamine Polyetherimide (Sibrid™, 15% N-methyl pyrrolidone) where the Sibrid™ (15%)/PS (20%) composition was varied at 50/50, 60/40 and 25/75 ratios.
#2 Mixture of PS (MW 350,000, ∼20% in THF) and thermally cross-linkable silicone system Gelest OE43 part A and part B, where the final silicone composition was varied from 5-10%.
#3 Mixture of PS (MW 350,000, ∼20%in THF) and aminofunctional copolymers of PDMS, where the final silicone composition was varied from 5-10%.

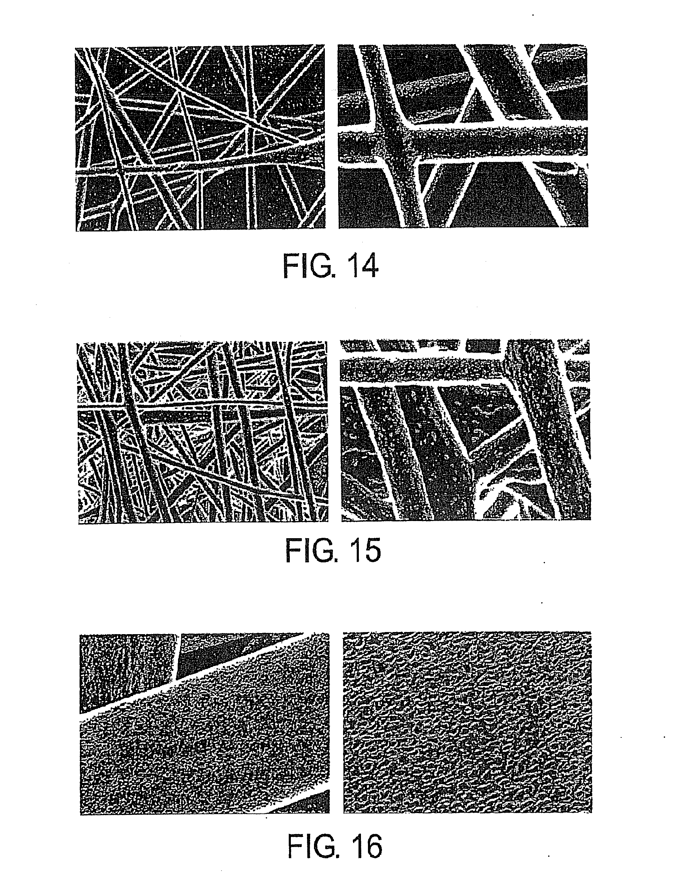

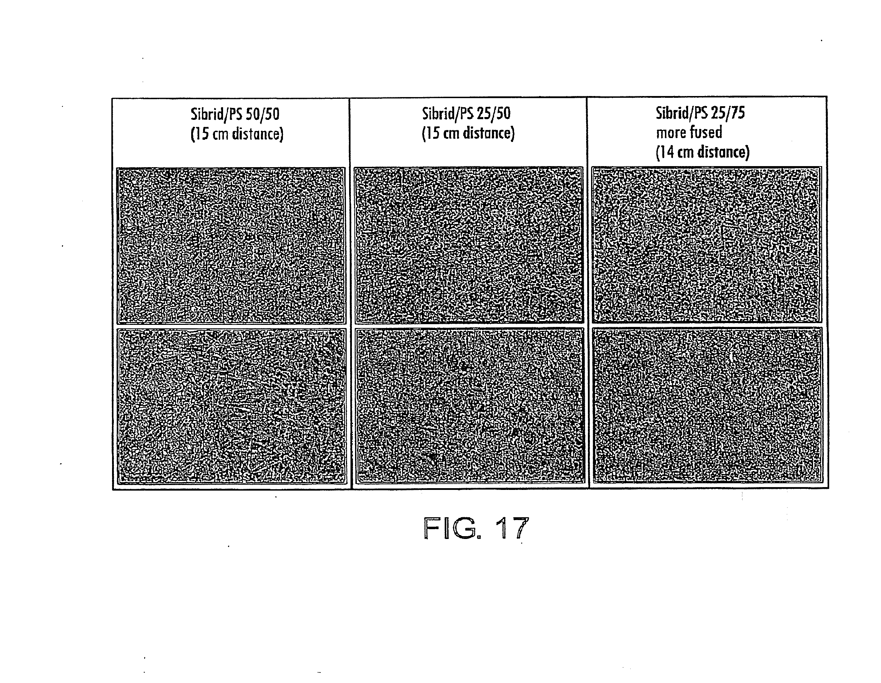

Figures 14-16 show typical nanofiber diameters for these systems (-900 nm-2 µm). These high resolution SEMs also depict the surface and inner nanofiber morphology of the nanofibers. Figure 17 shows optical micrographs of dense nanofiber mats that are typically used for cell culture experiments.

Electrospinning of different types of silicones result in unique morphologies, chemistries surface energies, and moduli. Different silicones will result in different chemistries on the surface even though these compositions are used as a blend. ATR-FTIR was used to characterize the samples for #2. Data show that the silicone is predominantly on the surface of the nanofibers even though polystyrene was the majority phase in both compositions. PDMS surface tension (γ) is 19.9 mN/m and PS γ is 40.7 mN/m and the lower surface tension polymer as expected has segregated to the surface of the nanofiber.

Silicones generally tend to be lower surface energy materials and hence less wettable in water. Therefore, electrospun Sibrid™/PS mixtures of 50/50 or 34/66 were post-modified using N₂O plasma treatment (30 sec) to make the mats hydrophilic. These treated mats also underwent accelerated aging condition (52 degrees, 5 day) to ascertain the hydrophilicity of the surface.

The nanofiber mat morphology also could be changed by manipulating the electrospinning processing conditions. More fused (MF) vs. less fused (LF) nanofiber mats were obtained by lowering the tip to substrate distance for Sibrid™/PS 25/75 ratio (Figure 17). This resulted in larger fused nanofibers (up to 5-6 µm).

### Cell culture

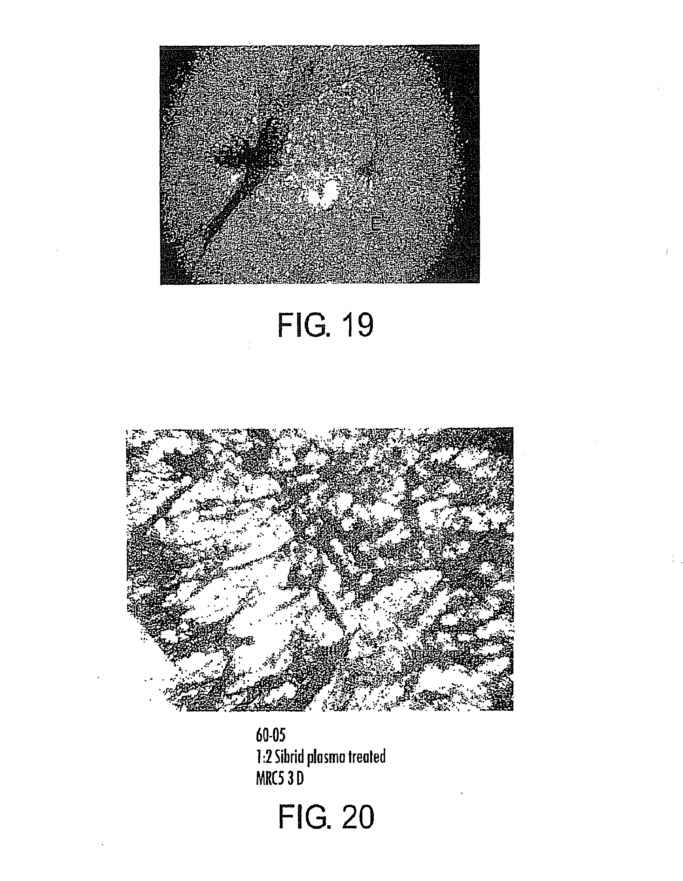

MRC5 fibroblast and HEPG2 liver cells were grown on silica/PVA and Sibrid™/PS systems. MRC5 cells were grown on silica/PVA and N₂O treated Sibird™/PS systems using tissue culture treated polystyrene (TCT) as a control surface. MRC5 cells attached and grew on both surfaces under standard cell culture conditions. On silica/PVA, MRC5 cells showed viable cell spreading after 24 hours. After 72 hours, morphology was distinct from that usually seen on PS. The MRC5 cells were very much spread out and had a pronounced leading edge (Figure 20). This has not been previously seen on TCT where the cells usually attach and grow in a flattened morphology. This could suggest enhanced cell function on silica/PVA surfaces over enhanced cell growth.

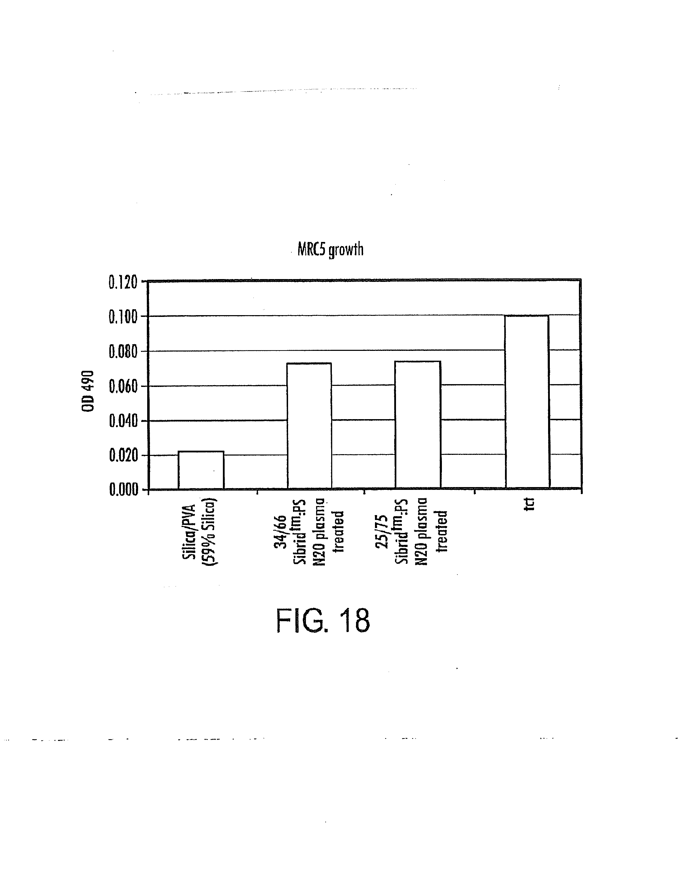

On N₂O treated Sibrid™/PS surfaces, MRC5 cells did not show any distinction compared to TCT. The cells were fibroblastic and looked similar to the ones on TCT (Figure 19). Figure 18 also shows relative MRC5 cell counts normalized to area of substrates. No contamination was noted for all the surfaces and the surfaces remained intact during the culture period.

Silica/PVA and Sibrid™/PS also support HEPG2 liver cell growth. Literature has shown that functional liver cells typically are spheroidal or rounded and grow slower than other cell types.

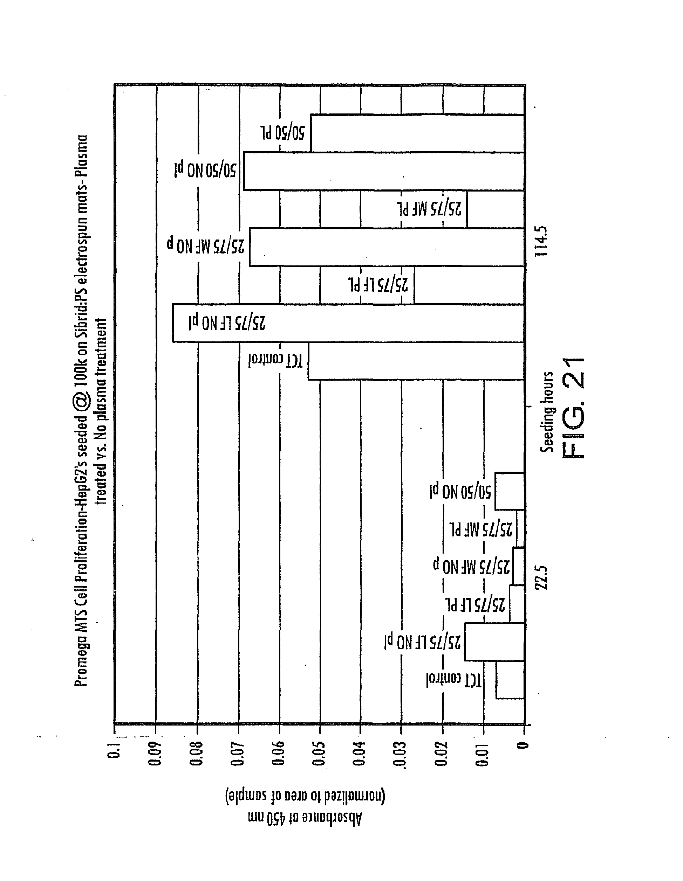

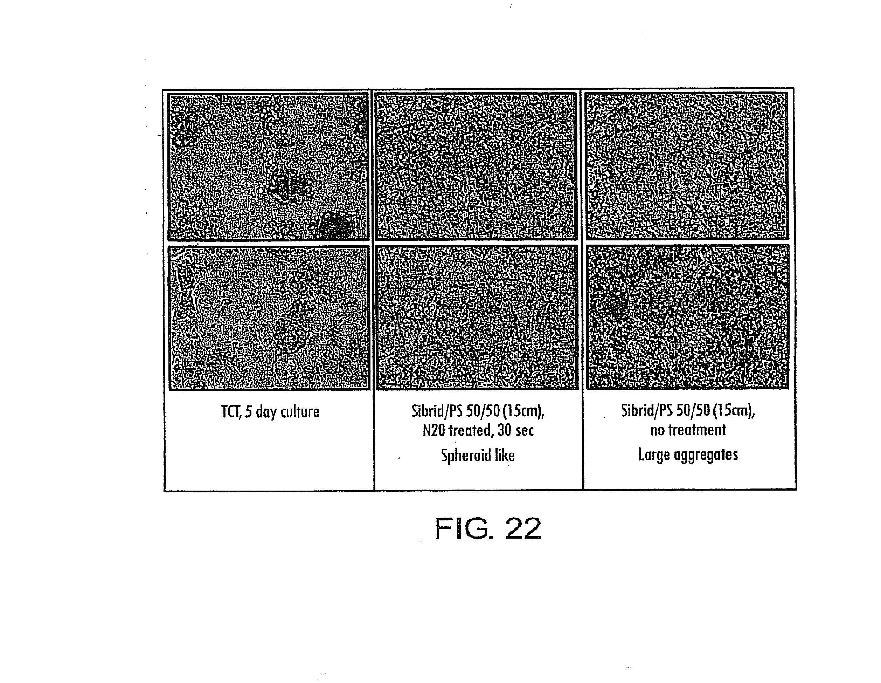

Significantly more cell growth was seen on the untreated Sibrid™/PS nanofiber surface at all compositions (50/50, 25/75) than on the TCT control surface after 5 days of standard cell culture. Figure 21 shows relative HEPG2 cell counts normalized to area of substrates after 1 day and 5 days of cell culture. These cells exhibited aggregates when grown on the Sibrid™/PS (Figure 22). Cells grew preferentially among and along the nanofibers, rather than in the flattened monolayer typically found on TCT. N₂O treated Sibrid™/PS showed lower cell counts compared to the untreated samples and TCT. Even though the cell growth was low on N₂O treated Sibrid™/PS, the cells remained in aggregates compared to flat and spread cells on TCT.

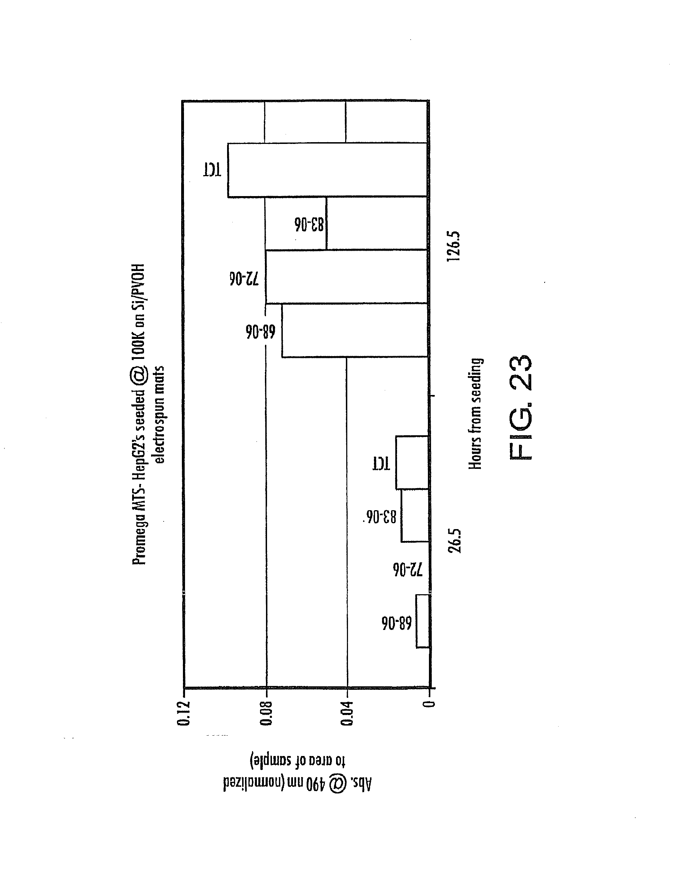

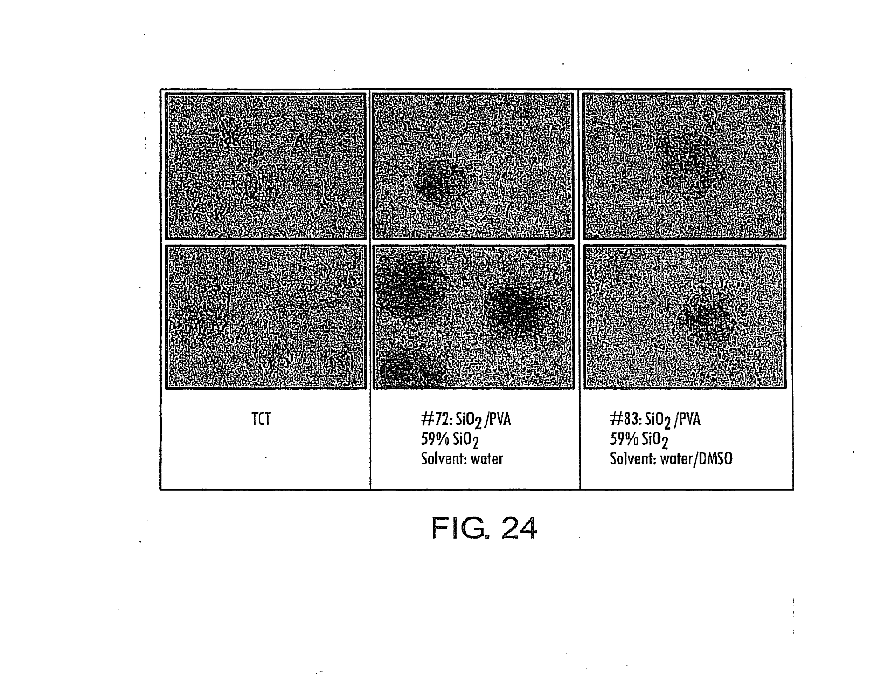

Comparable cell growth was seen on silica/PVA nanofiber surfaces to TCT control surface. Figure 23 shows relative HEPG2 cell counts normalized to area of substrates after 1 day and 5 days of cell culture. The cells on these surfaces also exhibited aggregates when compared to flattened morphology observed on TCT (Figure 24). Liver cell growth on day 5 was significantly high for silica/PVA (-DMSO) (83-06 in Figure 23) compared to fibers prepared in the absence of DMSO (68-06 and 72-06). The aggregates observed for this composition seem to lie on a monolayer of liver cells compared to spheroids observed for silica/PVA (+DMSO) that seem to aggregate directly on the substrate.

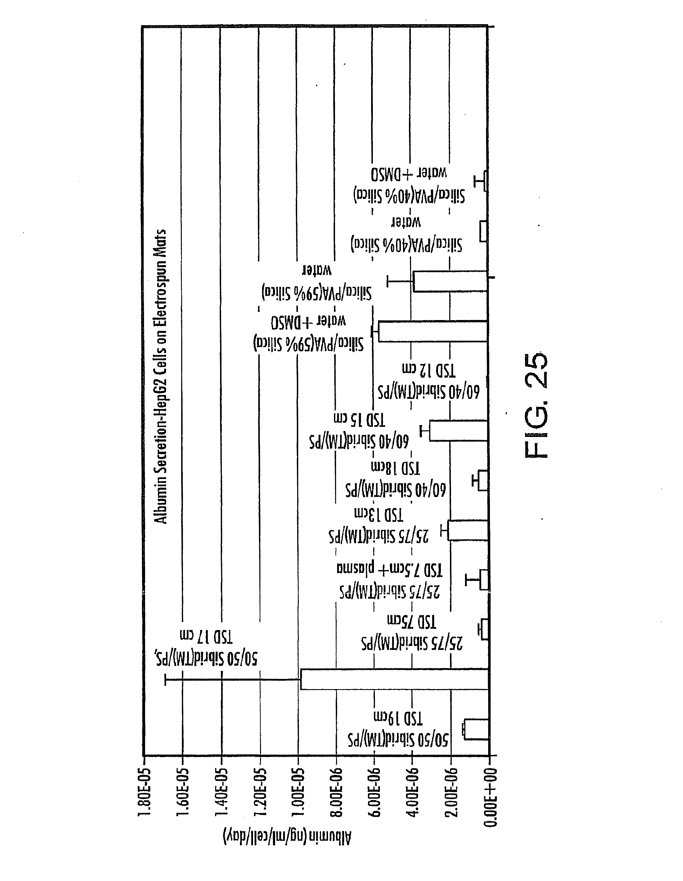

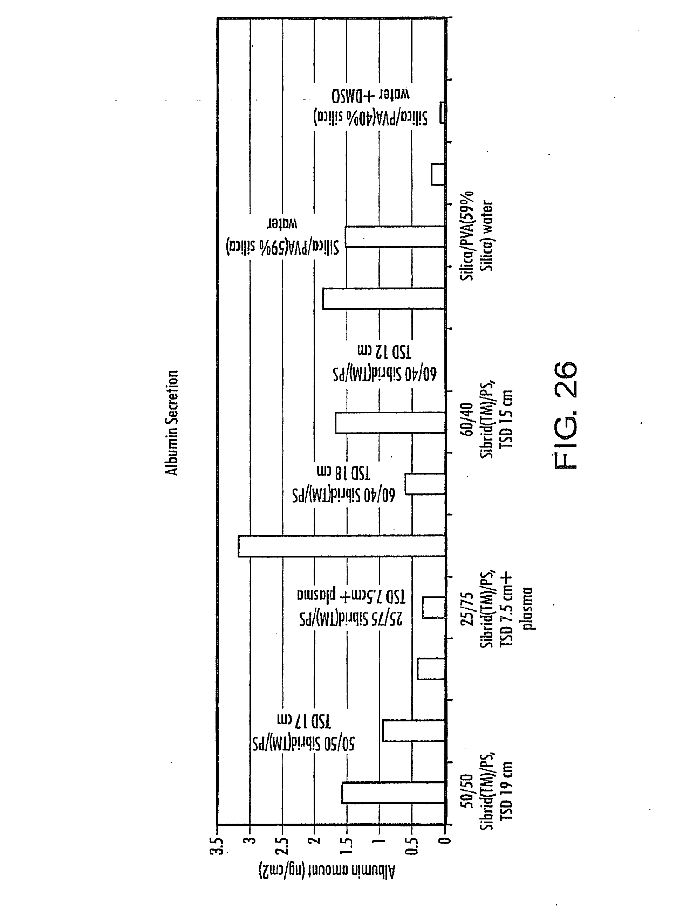

Figure 25 shows albumin production as a function of substrate and Figure 26 shows albumin production as a function of substrate normalized for cell count determined at the same time as albumin was assayed. All protocols for HepG2 proliferation and albumin secretion analysis are detailed in Example 1. HepG2 cell seeding density was kept at 100,000 cells in 3 ml of media for all experimental substrates.

### EXAMPLE 4-Relationship of Nanofiber Diameter and Composition to Cell Growth and Function

An investigation into the relationship between electrospun metal oxide / polymer nanofiber diameter and composition indicated that the inclusion of titanium into polystyrene nanofibers encourages more cell attachment and growth than polystyrene nanofibers of similar dimensions alone. Nanofibers composed of polystyrene and polystyrene/titania having nominal diameters of approximately 300 nm, 1,000 nm and 5,000 nm were electrospun onto glass slides and evaluated for growth and function of HepG2 hepatocytes (ATCC HB065). These cells produce proteins in amounts easily quantified and are considered a useful model for hepatocyte function. All results were repeated for confirmation.

Cells were maintained in IMDM/10%FBS/ antibiotic/antimycotic under standard culture conditions and used at passages 3-5. The seeding density was 20,000 cells/cm² for all samples and medium was changed every other day. Prior to assay for protein production, serum-containing medium was replaced by serum-free medium (IMDM containing ITES and antibiotic/antimycotic). Samples were rinsed gently three times with DPBS with calcium and magnesium to remove traces of albumin before the addition of serum free medium. Cells were allowed to grow for 24 hours before the serum free medium was assayed for protein production. Cell number was also determined at this time (CellTiter96, Promega). Total protein was assayed using the method of Hoefelschweiger (ProStain, Active Motif North America).

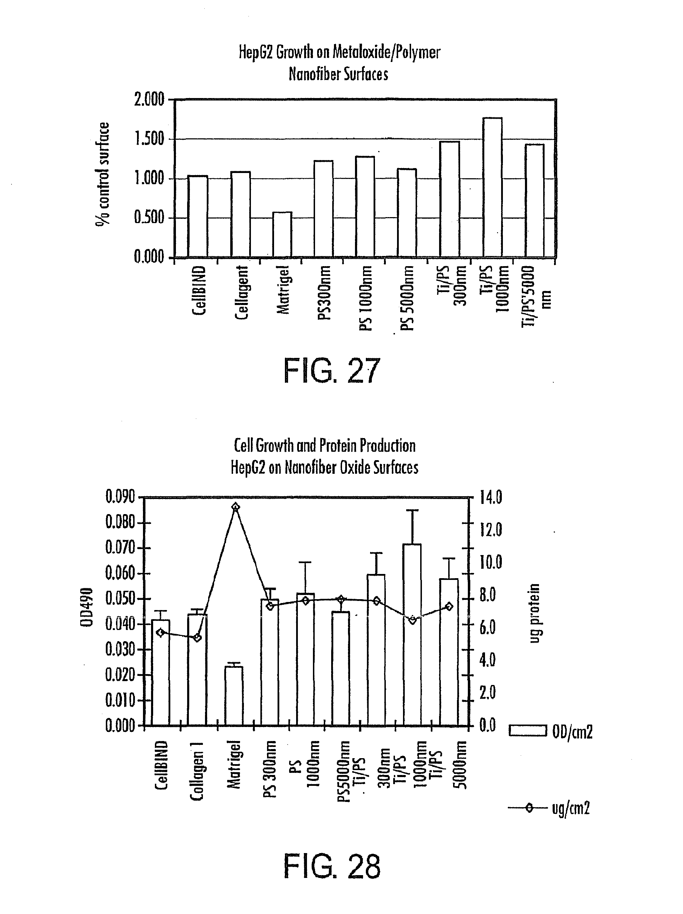

Figure 27 shows cell growth results on several nanofiber surfaces. Cell growth was significantly enhanced on the titania-containing nanofiber surfaces over tissue cultured on polystyrene, collagen and Matrigel as well as nanofibers made of polystyrene alone. On the larger diameter nanofibers, cells attached and grew like beads on a string, forming large clusters; on 1,000 nm or smaller nanofibers, small spheroids formed. Spheroid formation in cultured hepatocytes is also considered to indicate more *in vivo*-like behavior.

Protein production by hepatocytes is considered an indicator of normal cell function. Total protein excreted into the growth medium over a 24-hour period was measured in order to determine the degree of function of these cells on these surfaces. It is desirable in many applications to promote the function of cells, as is found *in vivo.* Figure 28 shows cell growth and protein production on several nanofiber surfaces. While no surface produced the degree of protein production as seen with Matrigel, which is a 3-dimensional protein gel coating, significantly more protein production (approx.150% greater) occurred on the 3-dimensional nanofiber surfaces than on standard polystyrene or collagen-coated surfaces. It is thought that the nanofibrous surfaces mimics in some respects the shape of cell matrix proteins. It is also unusual that surfaces that promote cell growth also allow enhanced differentiated function. The nanofiber surfaces appear to promote more differentiated behavior than standard culture surfaces.

Throughout this application, various publications are referenced. The disclosures of these publications in their entireties are hereby incorporated by reference into this application in order to more fully describe the compounds, compositions and methods described herein.

Various modifications and variations can be made to the materials, methods, and articles described herein. Other aspects of the materials, methods, and articles described herein will be apparent from consideration of the specification and practice of the materials, methods, and articles disclosed herein. It is intended that the specification and examples be considered as exemplary.

Embodiments of the invention include the following:
[1] A composition for making a nanofiber comprising:
   (a) a metal oxide, a metal oxide precursor, or a combination of metal oxide and metal oxide precursor and
   (b) a first solvent and second solvent,
   wherein the metal oxide, metal oxide precursor or combination of metal oxide and metal oxide precursor is soluble in the first solvent, the second solvent, or both the first and second solvent and wherein the first solvent and second solvent are immiscible.
[2] The composition of [1] above, wherein component (a) is a metal oxide and wherein the metal oxide comprises niobium oxide, tantalum oxide, titanium oxide, tungsten oxide, zirconium oxide, molybdenum oxide, vanadium pentoxide, nickel oxide, iron oxide, lead oxide, germanium oxide, manganese oxide, cobalt oxide, tin oxide, or a combination of two or more thereof.
[3] The composition of [1] above, wherein component (a) is a metal oxide precursor and wherein the metal oxide precursor comprises a metal salt, a metal alkoxide, a metal hydroxide, a metal ester, a metal nitride, a metal carbide, a metal halide, a metal sulfide, a metal selenide, a metal phosphate, a metal sulfate, a metal carbonate, a metal nitrate, a metal nitrite, a silsesquioxane, a silicone, silica, or a combination of two or more thereof.
[4] The composition of [1] above, wherein the composition further comprises a polymer, one or more polymer precursor, or a combination of two or more thereof.
[5] The composition of [4] above, wherein the polymer comprises polystyrene, a polyacrylate, a polyethylene, a polyimide, a polyether, a polysulfone, a polystyrenesulfonic acid, a polyethyleneimine, a polyvinyl alcohol, a polyvinylformal, a polyoxazaline, a polyvinylpyridine, a protein, an oligonucleotide, a polysaccharide, a polyamide, a polyvinylalkylether, a cycloolefinic copolymer, a polymethylmethacrylate, a polyester, a polymethacrylate, a phenolic compound, an epoxy compound, a urethane, a styrenic polymer, maleic anhydrides, a polypropylene oxides, a polyethylene oxide, a polyolefin, a polycarbonate, a fluoropolymer, a peptides, a cellulosic polymer, a hydrogel, polylysine, a polylactic acid, polylactide-co-glycolide, an alginate, a polycaprolactone, a polyorganosilsesquioxane, an acrylamide, a polysulfonate, a polyketone, a polyacrylonitrile, a polymethylpentene, a block co-polymer, a polyvinylpyrrolidine, a protein, a peptide, an oligonucleotide, or a combination of two or more thereof.
[6] The composition of [4] above, wherein the composition comprises a polymer and a metal oxide precursor, and wherein the polymer comprises polystyrene and the metal oxide precursor comprises a titanium compound, a niobium compound, or a tantalum compound.
[7] The composition of [4] above, wherein metal oxide, metal oxide precursor or combination of metal oxide and metal oxide precursor is soluble in the first solvent and the polymer is soluble in the second solvent, and the first solvent has a boiling point greater than the second solvent.
[8] The composition of [4] above, wherein the metal oxide, metal oxide precursor or combination of metal oxide and metal oxide precursor are soluble in the first solvent and the polymer is soluble in the second solvent, and the first solvent has a boiling point less than the second solvent.
[9] The composition of [1] above, wherein the first and second solvents have a difference in boiling points of at least 10 °C, measured at atmospheric pressure.
[10] The composition of [1] above, wherein the first and second solvents have a difference in vapor pressures of at least 10 mm Hg at 20 °C.
[11] The composition of [1] above, wherein the first and second solvent are selected from the group consisting of acetone, acetonitrile, carbon tetrachloride, chloroform, cyclohexane, 1,2-dichloroethane, dichloromethane, diethyl ether, dimethyl formamide, dimethyl sulfoxide, 1,4-dioxane, ethanol, ethyl acetate, heptane, hexane, methanol, methyl-tert-butyl ether, pentane, 1-propanol, 2-propanol, tetrahydrofuran, toluene, 2,2,4-trimethylpentane, water, ethanol, benzene, butanol, methyl ethyl ketone, N-methyl pyrrolidine, dimethylacetamide, formic acid, acetic acid, citric acid, and a combination of two or more thereof.
[12] The composition of [1] above, wherein the composition further comprises a catalyst, bioactive agent, a metallic or organic conductive material, a magnetic material, a piezoelectric material, a ferroelectric material, a dielectric material, a radioactive material, a phosphorescent material, a dye, a surfactant, or a rare earth metal.
[13] A fiber-forming composition comprising:
   (g) a metal oxide, a metal oxide precursor or a combination of a metal oxide and a metal oxide precursor;
   (h) a polymer or a polymer precursor; and
   (i) a first solvent and second solvent,
   wherein the metal oxide, metal oxide precursor, combination of metal oxide and metal oxide precursor, polymer or polymer precursor are soluble in the first solvent, the second solvent, or both the first and second solvent, and the first solvent and second solvent are immiscible.
[14] The composition of [13] above, wherein the component of (a) comprises a metal oxide and wherein the metal oxide comprises niobium oxide, tantalum oxide, titanium oxide, tungsten oxide, zirconium oxide, molybdenum oxide, vanadium pentoxide, a nickel oxide, an iron oxide, a lead oxide, a germanium oxide, a manganese oxide, a cobalt oxide, a tin oxide, or a combination of two or more thereof.
[15] The composition of [13] above, wherein the component of (a) comprises a metal oxide precursor comprising a metal salt, a metal alkoxide, a metal hydroxide, a metal ester, a metal nitride, a metal carbide, a metal halide, a metal sulfide, a metal selenide, a metal phosphate, a metal sulfate, a metal carbonate, a metal nitrate, a metal nitrite, a silsesquioxane, a silicone, silica, or a combination of two or more thereof.
[16] The composition of [13] above, wherein the component of (a) is a metal oxide precursor and wherein the metal oxide precursor comprises a niobium compound, a tantalum compound, a titanium compound, an aluminum compound, a silicon compound, a cerium compound, a calcium compound, a cadmium compound, an erbium compound, a selenium compound, a tellurium compound, a gallium compound, an arsenic compound, a germanium compound, a zinc compound, a tin compound, an indium compound, a ruthenium compound, a rhenium compound, a nickel compound, a tungsten compound, a molybdenum compound, a magnesium compound, or a combination of two or more thereof.
[17] The composition of [14] above, wherein the metal oxide precursor, the polymer, the polymer precursor, or any combination thereof produces one or more co-solvents *in situ.*
[18] The composition of [14] above, wherein the polymer comprises polystyrene, a polyacrylate, a polyethylene, a polyimide, a polyether, a polysulfone, a polystyrenesulfonic acid, a polyethyleneimine, a polyvinyl alcohol, a polyvinylformal, a polyoxazaline, a polyvinylpyridine, a protein, an oligonucleotide, a polysaccharide, a polyamide, a polyvinylalkylether, a cycloolefinic copolymer, a polymethylmethacrylate, a polyester, a polymethacrylate, a phenolic compound, an epoxy compound, a urethane, a styrenic polymer, maleic anhydrides, a polypropylene oxides, a polyethylene oxide, a polyolefin, a polycarbonate, a fluoropolymer, a peptides, a cellulosic polymer, a hydrogel, polylysine, a polylactic acid, polylactide-co-glycolide, an alginate, a polycaprolactone, a polyorganosilsesquioxane, an acrylamide, a polysulfonate, a polyketone, a polyacrylonitrile, a polymethylpentene, a block co-polymer, a polyvinylpyrrolidine, a protein, a peptide, an oligonucleotide, or a combination of two or more thereof.
[19] The composition of [14] above, wherein the composition comprises a polymer and metal oxide precursor, wherein the polymer comprises polystyrene and the metal oxide precursor comprises a titanium compound, a niobium compound, or a tantalum compound.
[20] The composition of [14] above, wherein the metal oxide precursor is soluble in the first solvent and the polymer is soluble in the second solvent, and the first solvent has a boiling point greater than the second solvent.
[21] The composition of [14] above, wherein the metal oxide precursor is soluble in the first solvent and the polymer is soluble in the second solvent, and the first solvent has a boiling point less than the second solvent.
[22] The composition of [14] above, wherein the first and second solvents have a difference in boiling points of at least 10 °C measured at atmospheric pressure.
[23] The composition of [14] above, wherein the first and second solvents have a difference in vapor pressures of at least 10 mm Hg at 20 °C.
[24] The composition of [14] above, wherein the first and second solvent are selected from the group consisting of acetone, acetonitrile, carbon tetrachloride, chloroform, cyclohexane, 1,2-dichloroethane, dichloromethane, diethyl ether, dimethyl formamide, dimethyl sulfoxide, 1,4-dioxane, ethanol, ethyl acetate, heptane, hexane, methanol, methyl-tert-butyl ether, pentane, 1-propanol, 2-propanol, tetrahydrofuran, toluene, 2,2,4-trimethylpentane, water, ethanol, benzene, butanol, methyl ethyl ketone, N-methyl pyrrolidine, dimethylacetamide, formic acid, acetic acid, citric acid, and a combination of two or more thereof.
[25] The composition of [14] above, wherein the composition further comprises a catalyst, bioactive agent, a metallic or organic conductive material, a magnetic material, a piezoelectric material, a ferroelectric material, a dielectric material, a radioactive material, a phosphorescent material, a dye, a surfactant or a rare earth metal.

## Claims

1. A substrate for immobilizing cells or tissue comprising:
(a) a network of nanofibers; and
(b) a base substrate comprising a non-woven or woven porous or non-porous substrate, wherein the base substrate comprises a first outer surface, wherein the network of nanofibers is adjacent to the first outer surface of the base substrate. wherein the nanofibers in the network of nanofibers comprise;
(i) polystyrene and niobium oxide, tantalum oxide, titanium oxide or any combination thereof; or
(ii) polyvinyl alcohol and silica; or
(iii) polystyrene and silica or silicone.

2. The substrate of claim 1, in which the base substrate is porous.
